# FASCICULE DE BREVET EUROPEEN

(11) **EP 1 979 339 B1**
(45) Date de publication et mention de la délivrance du brevet: **31.10.2012**
(21) Numéro de dépôt: 07718115.4
(22) Date de dépôt: 17.01.2007
(51) Int. Cl.: C07D 401/06, C07D 401/14, C07D 403/06, C07D 413/14, A61K 31/4166

(54) **NOUVEAUX DERIVES D'UREE CYCLIQUE, LEUR PREPARATION ET LEUR UTILISATION PHARMACEUTIQUE COMME INHIBITEURS DE KINASES**
NEUE ZYKLISCHE HARNSTOFFDERIVATE, DEREN HERSTELLUNG UND PHARMAZEUTISCHE VERWENDUNG DAMIT ALS KINASE-INHIBITOREN
NOVEL CYCLIC UREA DERIVATIVES, PREPARATION THEREOF AND PHARMACEUTICAL USE THEREOF AS KINASE INHIBITORS

(30) Priorité: 23.01.2006 FR 0600567
(43) Date de publication de la demande: 15.10.2008
(73) Titulaire: Aventis Pharma S.A., 92160 Antony (FR)
(72) Inventeur: HALLEY, Frank, 92160 Antony (FR); EL-AHMAD, Youssef, 92160 Antony (FR); CERTAL, Victor, 92160 Antony (FR); STROBEL, Hartmut, 65926 Francfort (DE); RITTER, Kurt, 65926 Francfort (DE); RUF, Sven, 65926 Francfort (DE); DAGALLIER, Anne, 92160 Antony (FR); VENOT, Corinne, 92160 Antony (FR)
(74) Mandataire: Sartorius, Jérome
(86) Numéro de dépôt international: PCT/FR2007/000079
(87) Numéro de publication internationale: WO 2007/083017

(56) Documents cités:
- EP-A- 0 494 819
- WO-A-2004/070050
- WO-A-2005/049580

## Description

La présente invention concerne de nouveaux dérivés d'urée cyclique, leur procédé de préparation, leur application comme médicaments, les compositions pharmaceutiques les renfermant et l'utilisation pharmaceutique de tels dérivés pour la prévention et le traitement d'affections capables d'être modulées par l'inhibition de l'activité de protéines kinases.

La présente invention concerne de nouveaux dérivés d'urée cyclique possédant des effets inhibiteurs pour des protéines kinases.

Les produits de la présente invention peuvent ainsi notamment être utilisés pour la prévention ou le traitement d'affections capables d'être modulées par l'inhibition de l'activité de protéines kinases.

L'inhibition et la régulation de protéines kinases constituent notamment un nouveau puissant mécanisme d'action pour le traitement d'un grand nombre de tumeurs solides ou liquides.

De telles affections que peuvent traiter les produits de la présente demande sont donc tout particulièrement les tumeurs solides ou liquides.

De telles protéines kinases appartiennent notamment au groupe suivant: EGFR, Fak, FLK-1, FGFR1, FGPR2, FGFR3, FGFR4, FGFR5, flt-1, IGF-1R, KDR, PLK, PDGFR, tie2, VEGFR, AKT, Raf.

On indique tout particulièrement la protéine kinase IGF1-R (Insulin Growth Factor-1 Receptor).

La présente invention concerne ainsi particulièrement de nouveaux inhibiteurs du récepteur IGF-1R qui peuvent être utilisés pour des traitements en oncologie.

Le cancer reste une maladie pour laquelle les traitements existants sont clairement insuffisants. Certaines protéines kinases dont notamment IGF-1R (Insulin Growth Factor 1 Receptor)jouent un rôle important dans de nombreux cancers. L'inhibition de telles protéines kinases est potentiellement importante dans la chimiothérapie de cancers notamment pour supprimer la croissance ou la survie de tumeurs. La présente invention concerne donc l'identification de nouveaux produits qui inhibent de telles protéines kinases.

Les protéines kinases participent aux événements de signalisation qui contrôlent l'activation, la croissance et la différentiation des cellules en réponse, soit à des médiateurs extracellulaires, soit à des changements de l'environnement. En générale, ces kinases appartiennent à deux groupes: celles qui phosphorylent préférentiellement les résidus serines et/ou thréonine et celles qui phosphorylent préférentiellement les résidus tyrosines [S.K.Hanks and T.Hunter, FASEB. J., 1995, 9, pages 576-596]. Les sérine/thréonine kinases sont par exemple, les isoformes des protéines kinases C [A.C.Newton, J. Biol. Chem., 1995, 270, pages 28495-28498] et un groupe de kinases dépendantes des cyclines, comme cdc2 [J.Pines, Trends in Biochemical Sciences, 1995, 18, pages 195-197]. Les tyrosine kinases comprennent les récepteurs aux facteurs de croissance comme le récepteur au facteur de croissance épidermal (EGF) [S.Iwashita and M.Kobayashi, Cellular Signalling, 1992, 4, pages 123-132], et des kinases cytosoliques comme p56tck, p59fYn, ZAP-70 et les kinases csk [C. Chan et. al., Ann. Rev. Immunol., 1994, 12, pages 555-592].

Des niveaux anormalement élevés d'activité protéine kinase ont été impliqués dans de nombreuses maladies, résultant de fonctions cellulaires anormales. Ceci peut prévenir soit directement soit indirectement, d'un disfonctionnement dans les mécanismes de contrôle de l'activité kinase, lié par exemple à une mutation, une sur-expression ou une activation inapproprié de l'enzyme, ou par une sur- ou sous-production de cytokines ou des facteurs de croissance, également impliqués dans la transduction des signaux en amont ou en aval des kinases. Dans tous ces cas, une inhibition sélective de l'action des kinases laisse espérer un effet bénéfique.

Le récepteur de type 1 pour l'insulin-like growth factor (IGF-I-R) est un récepteur transmembranaire à activité tyrosine kinase qui se lie en premier lieu à l'IGFI mais aussi à l'IGFII et à l'insuline avec une plus faible affinité. La liaison de l'IGF1 à son récepteur entraîne une oligomérisation du récepteur, l'activation de la tyrosine kinase, l'autophosphorylation intermoléculaire et la phosphorylation de substrats cellulaires (principaux substrats : IRS1 et Shc). Le récepteur activé par son ligand induit une activité mitogènique dans les cellules normales. Cependant IGF-I-R joue un rôle important dans la croissance dite anormale.

Plusieurs rapports cliniques soulignent le rôle important de la voie IGF-I dans le développement des cancers humains :
IGF-I-R est souvent trouvé sur-exprimé dans de nombreux types tumoraux (sein, colon, poumon, sarcome, prostate, myelome multiple -) et sa présence est souvent associée à un phénotype plus agressif.

De fortes concentrations d'IGF1 circulant sont fortement corrélées à un risque de cancer de la prostate, poumon et sein.

De plus, il a été largement documenté que IGF-I-R est nécessaire à l'établissement et au maintient du phénotype transformé in vitro comme in vivo[Baserga R, Exp. Cell. Res., 1999, 253, pages 1-6]. L'activité kinase d'IGF-I-R est essentielle à l'activité de transformation de plusieurs oncogènes: EGFR, PDGFR, l'antigène grand T du virus SV40, Ras activé, Raf, et v-Src. L'expression d'IGF-I-R dans des fibroblastes normaux induit un phénotype néoplasique, qui peut ensuite entraîner la formation de tumeur in vivo. L'expression d'IGF-I-R joue un rôle important dans la croissance indépendante du substrat. IGF-I-R a également été montré comme un protecteur dans l'apoptose induite par chimiothérapie-, radiation-, et l'apoptose induite par des cytokines. De plus, l'inhibition d'IGF-I-R endogène par un dominant négatif, la formation de triple hélice ou l'expression d'un antisens provoque une suppression de l'activité transformante *in vitro* et la diminution de la croissance de tumeurs dans les modèles animaux.

Parmi les kinases pour lesquelles une modulation de l'activité est recherchée, FAK (Focal Adhesion Kinase) est également une kinase préférée.

FAK est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33) : 23439-442 (1992)]. L'autophosphorylation sur la tyrosine 397 de FAK représente un site de liaison pour une autre tyrosine kinase, Src, via son domaine SH2 [Schaller et al. Mol. Cell. Biol. 14 : 1680-1688 1994; Xing et al. Mol. Cell. Biol. 5 : 413-421 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372 : 786-791 1994; Schlaepfer et al. Prog. Biophy. Mol. Biol. 71 : 435-478 1999; Schlaepfer and Hunter, J. Biol. Chem. 272 : 13189-13195 1997].

L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 : 1461-1469 1999]. Phosphatidylinositol-3-OH kinase (PI3-kinase) se lie aussi à FAK sur la tyrosine 397 et cette interaction pourrait être nécessaire à l'activation de PI3-kinase [Chen and Guan, Proc. Nat. Acad. Sci. USA. 91 : 10148-10152 1994; Ling et al. J. Cell. Biochem. 73 : 533-544 1999]. Le complexe FAK/Src phosphoryle différents substrats comme la paxilline et p130CAS dans les fibroblastes. [Vuori et al. Mol. Cell. Biol. 16 : 2606-2613 1996].

Les résultats de nombreuses études soutiennent l'hypothèse que les inhibiteurs de FAK pourraient être utiles dans le traitement du cancer. Des études ont suggéré que FAK pourrait jouer un rôle important dans la prolifération et/ou la survie cellulaire in vitro. Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143 : 1997-2008 1998]. D'autres auteurs ont montré que des cellules tumorales traitées avec des oligonucléotides anti-sens de FAK perdent leur adhésion et entrent en apoptose (Xu et al, Cell Growth Differ. 4 : 413-418 1996). Il a également été démontré que FAK promeut la migration des cellules in vitro. Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112 : 2677-91 1999]. La surexpression du domaine C-terminal de FAX (FRNK) bloque l'étirement des cellules adhérentes et réduit la migration cellulaires in vitro [Richardson A. and Parsons J.T. Nature. 380 : 538-540 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires in vitro, suggère le rôle potentiel de FAK dans les processus néoplasiques. Une étude récente a effectivement démontré l'augmentation de la prolifération des cellules tumorales in vivo après induction de l'expression de FAK dans des cellules d'astrocytome humain [Cary L.A. et al. J. Cell Sci. 109 : 1787-94 1996; Wang D et al. J. Cell Sci. 113 : 4221-4230 2000]. De plus, des études immunohistochimiques de biopsies humaines ont démontré que FAK était surexprimé dans les cancers de la prostate, du sein, de la thyroide, du colon, du mélanome, du cerveau et du poumon, le niveau d'expression de FAK étant directement corrélé aux tumeurs présentant le phénotype le plus agressif [Weiner TM, et al. Lancet. 342 (8878) : 1024-1025 1993; Owens et al. Cancer Research. 55 : 2752-2755 1995; Maung K. et al. Oncogene 18 : 6824-6828 1999; Wang D et al. J. Cell Sci. 113 : 4221-4230 2000].

La protéine kinase AKT (également connue sous le nom de PKB) et la phosphoinositide 3-kinase (PI3K) sont impliqués dans une voie de signalisation cellulaire qui transmet des signaux venant de facteurs de croissance activant des récepteurs membranaires.

Cette voie de transduction est impliquée dans de multiples fonctions cellulaires: régulation de l'apoptose, contrôle de la transcription et de la traduction, métabolisme du glucose, angiogénèse et intégrité mitochondriale. Identifié d'abord comme un acteur important dans les voies de signalisation insulino-dépendantes régulant des réponses métaboliques, la sérine/thréonine kinase AKT a ensuite été identifiée comme un médiateur jouant un rôle clé dans la survie induite par des facteurs de croissance. Il a été montré qu'AKT pouvait inhiber la mort par apoptose induite par des stimuli variés, dans un certain nombre de types cellulaires et de cellules tumorales. En accord avec ces constatations, il a été montré qu'AKT pouvait, par phosphorylation de résidus sérine donnés, inactiver BAD, GSK3β, caspase-9, le facteur de transcription Forkhead et activer IKKalpha et e-NOS. Il est intéressant de noter que la protéine BAD est retrouvée hyper-phosphorylée dans 11 lignées cellulaires humaines tumorales sur 41 étudiées. De plus, il a été montré que l'hypoxie modulait l'induction du VEGF dans des cellules transformées par Ha-ras en activant la voie PI3K/AKT et en impliquant la séquence de fixation du facteur de transcription HIF-1 (hypoxia inducible factor-1) appelé HRE pour «hypoxy-responsive-element ».

AKT joue un rôle très important dans les pathologies cancéreuses. L'amplification et/ou la surexpression d'AKT a été rapportée dans de nombreuses tumeurs humaines comme le carcinome gastrique (amplification d'AKT1), les carcinomes de l'ovaire, du sein ou du pancréas (amplification et surexpression d'AKT2) et les carcinomes du sein déficients en récepteurs aux oestrogènes ainsi que les carcinomes de la prostate indépendants des androgènes (surexpression d'AKT3). De plus, AKT est activée constitutivement dans toutes les tumeurs PTEN (-/-), la phosphatase PTEN étant délétée ou inactivée par des mutations dans de nombreux types de tumeurs comme les carcinomes de l'ovaire, de la prostate, de l'endomètre, les glioblastomes et les mélanomes. AKT est également impliqué dans l'activation oncogénique de bcr-abl (Références : Khawaja A., Nature 1999, 401, 33-34; Cardone et al. Nature 1998, 282, 1318-1321; Kitada S. et al., Am J Pathol 1998 Jan; 152(1) : 51-61; Mazure NM et al. Blood 1997, 90, 3322-3331; Zhong H. et al. Cancer Res. 2000, 60, 1541-1545).

WO 2004/070050 divulgue des composés inhibiteurs de proteines kinases.

La présente invention a pour objet les produits de formule (Ia) : 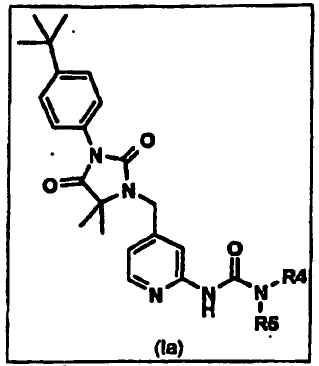 dans laquelle les radicaux NR4R5 sont choisis parmi les radicaux suivants nommés ex 9 à ex 31: 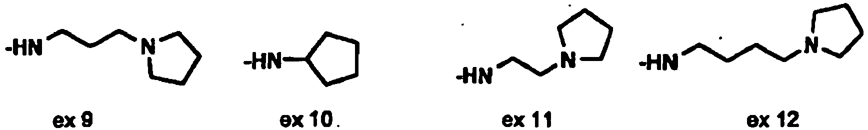 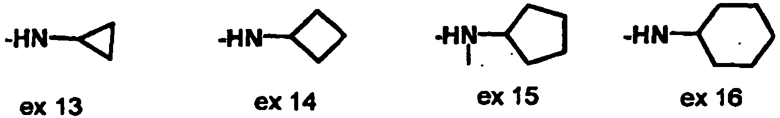 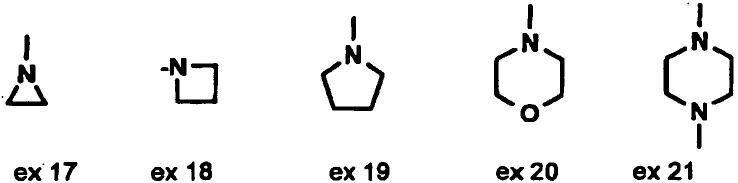 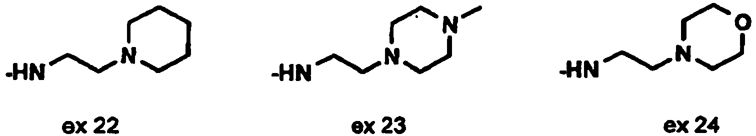 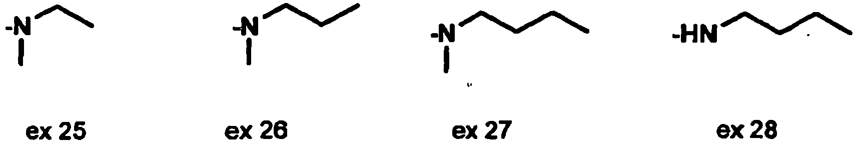 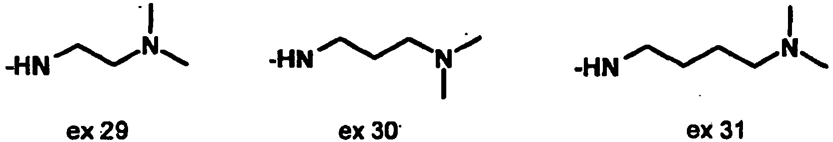 lesdits produits de formule (Ia) étant sous toutes les formes isomères possibles racémiques, énantiomères et diastéréo-isomères, ainsi que les sels d'addition avec les acides minéraux et organiques ou avec les bases minérales et organiques desdits produits de formule (Ia).

Lorsque les produits de formule (Ia) comportent un radical amino salifiable par un acide il est bien entendu que ces sels d'acides font également partie de l'invention. On peut citer les sels fournis avec les acides chlorhydrique ou méthanesulfonique par exemple.

Les sels d'addition avec les acides minéraux ou organiques des produits de formule (Ia) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques.

On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomère est utilisé dans la présente demande dans son sens le plus large et concerne donc l'ensemble des composés indiqués ci-dessus.

Les produits de formule (Ia) selon la présente invention peuvent être préparés selon les méthodes usuelles connues de l'homme du métier.

Les produits de formule (Ia) selon la présente invention peuvent être préparés par l'application ou l'adaptation de méthodes connues et notamment des méthodes décrites dans la littérature comme par exemple celles décrites par R. C. Larock dans: Comprehensive Organic Transformations, VCH publishers, 1989.

Les produits selon la présente invention peuvent notamment être préparés comme indiqué dans les Schémas de synthèse décrits ci-après: Schéma de préparation d'intermédiaires et 1 Schéma Général de synthèse : le le Schéma Général 2 bis ci-dessous.

Les préparations des exemples de la présente invention donnent des illustrations des Schémas ci-dessous. De tels schémas de synthèse font partie de la présente invention : la présente invention a ainsi également pour objet les procédés de préparation des produite de formule (Ia) tels que définis dans le Schéma Général 2 bis, ci-dessous. Des intermédiaires peuvent être préparés selon le procédé défini ci-après. Schéma de préparation d'intermédiaires:

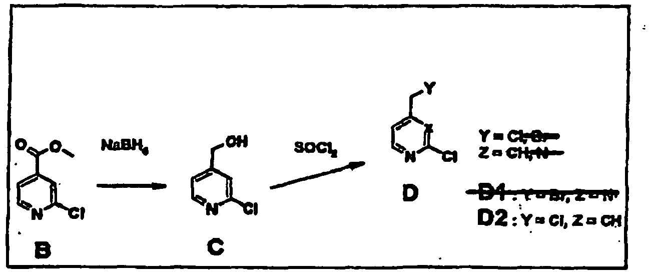

Dans le Schéma de préparation d'intermédiaires :

L'alcool C peut être préparé par réduction de l'ester B avec un agent de réduction tel que le borohydrure de sodium dans un solvent tel que l'ethanol à une température comprise entre 20°C et la température de reflux du solvent comme décrit para Zanka, A. et coll. (Synlett (1999), (10), 1636-1638).

Le produit D2 est préparé par chloration de l'alcool C comme dans les conditions décrites par Fucase K. et coll. (Tetrahedron Lett, 1991, 32(32), 4019-4022) par traitement avec du chlorure de thionyle en présence du DMF dans un solvant tel que le dichlorométhane à une température comprise entre 0°C et 20°C.

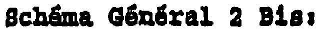

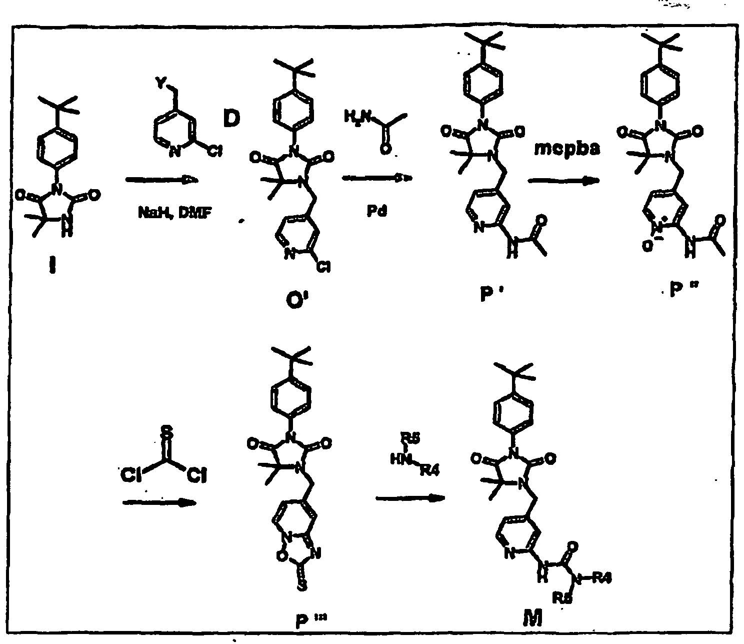

Le produit M peut également être préparé en suivant la synthèse décrite dans le Schéma Général 2 Bis:

Le produit O' peut être préparé par réaction des produits I et D avec de l'hydrure de sodium dans le terahydrofurane ou le N,N-dimethylformamide à une température comprise entre 0°C et 60°C tel que décrit par Johnson T.A. et coll. (J. Am.Chem.Soc. (2002), 124, 11689-11698).

Le produit P' peut être préparé à partir de O' par réaction avec l'acetamide (NH2COCH3) en présence d'un catalyseur à base de palladium tel que l'acétate. palladium et d'un ligand tel que le xantphos dans un solvent tel que le toluène, le dioxane ou le tert-butanol comme par exemple dans les conditions décrites par BUCHWALD, S. L. et coll. (J. Am. Chem. Soc. 2002, 124, 6043-6048).

Le produit P'' peut être préparé par oxydation de P' par un oxydant tel que l'acide meta chloroperbenzoique dans un souvent tel que le dichloromethane comme par exemple dans les conditions décrites par SOLLOGOUB, M. et coll. (Tet. Lett. 2002, 43 (17), 3121-3123).

Le produit P''' peut être obtenu en traitant le produit P'' avec du thiophosgène en présence de base telle que l'hydrogénocarbonate de sodium dans un solvent tel que l'éthanol comme par exemple dans les conditions décrites par ROUSSEAU, D. et Coll. (Can. J. Chem. 1977, 55, 3736-3739).

Le produit M peut être obtenu en traitant le produit P''' avec une amine NHR4R5 dans un solvent tel que le dioxanne ou le diméthylsulfoxide comme par exemple dans les conditions décrites par OHSAWA, A. et Coll. (Chem. Pharm. Bull. 1980, 28, 3570-3575).

Dans de telles préparations des produits de formule (Ia) selon la présente invention, les produits de départ, les intermédiaires et les produits de formule (Ia), qui peuvent être sous forme protégée, peuvent être soumis, si nécessaire ou si désiré, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation dé groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction de réduction des composés nitrés en composés aminée,
j) une réaction d'élimination des groupements protecteurs que peuvent porter les fonctions réactives protégées,
k) une réaction de salification par un acide minéral ou organique ou par une base pour obvenir le sel correspondant,
l) une réaction de dédoublement des formes racémiques en produits dédoublés,
lesdits produits de formule (Ia) ainsi obtenus étant sous toutes les formes isomères possibles racémiques, énantiomères et diastéréoisomères.

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus. Dans les réactions décrites ci-après, il peut être nécessaire de protéger des groupes fonctionnels réactifs tels que par exemple des groupements hydroxy, acyle, carboxy libres ou encore amino et monoalkylamino, imino, thio, .. qui peuvent donc être protégés par les groupements protecteur appropriés.

On peut utiliser des groupes protecteurs conventionnels en accord avec les pratiques usuelles standard comme ceux décrits par exemple par T.W. Greene and P.G.M.Wuts dans "Protective Groups in organic Chemistry" John Wiley and Sons, 1991.

La liste suivante, non exhaustive, d'exemples de protection de fonctions réactives peut être citée :
- les groupements hydroxyle peuvent être protégés par exemple par les radicaux alkyle tels que tert-butyle, triméthyl-silyle, tert-butyldiméthylsilyle, méthoxyméthyle, tétrahydro-pyrannyle, benzyle ou acétyle,
- les groupements amino peuvent être protégés par exemple par les radicaux acétyle, trityle, benzyle, tert-butoxycarbonyle, benzyloxycarbonyle, phtalimido ou d'autres radicaux connus dans la chimie des peptides,

- les groupements acyles tel que le groupement formyle peuvent être protégée par exemple sous forme de cétals ou de thiocétals cyclique ou non cycliques tels que le diméthyl ou diéthylcétal ou l'éthylène dioxycétal, ou le diéthylthiocétal ou l'éthylènedithiocétal,
- les fonctions acides des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante:
- les fonctions acides peuvent être protégées par exemple sous forme d'esters formés avec les esters facilement clivables tels que les esters benzyliques ou terbutyliques ou des esters connus dans la chimie des peptides.

Ces réactions a) à k) indiquées ci-dessus peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) Les éventuels groupements alkylthio des produits décrits ci-dessus, dans lesquels le radical alkyle est éventuellement substitué par un ou plusieurs atomes d'halogène, notamment de fluor, peuvent être, si désiré, transformés en les fonctions sulfoxyde ou sulfone correspondantes dans les conditions usuelles connues de l'homme du métier telles que par exemple par les peracides comme par exemple l'acide poracétique ou l'acide métachloroperbenzoique ou encore par l'ozone, l'oxone, le périodate de sodium dans un solvant tel que par exemple le chlorure de méthylène ou le dioxanne à la température ambiante.

L'obtention de la fonction sulfoxyde peut être favorisée par un mélange équimolaire du produit renfermant un groupement alkylthio et du réactif tel que notamment un peracide.

L'obtention de la fonction sulfone peut être favorisée par un mélange du produit renfermant un groupement alkylthio avec un excès du réactif tel que notamment un peracide
d) La réaction de transformation d'une fonction cétone en oxime peut être réalisée dans les conditions usuelles connues de l'homme de métier, telle que notamment une action en présence d'une hydroxylamine éventuellement O-substituée dans un alcool tel que par exemple l' éthanol, à température ambiante ou en chauffant.
e) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique.

Les éventuelles fonctions carboxy libre des produits décrits ci-dessus peuvent être, si désiré, réduites en fonction alcool notamment par de l'hydrure de bore.
f) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhy-drique dans de l'eau ou de l'acide trifluoroacétique au reflux.
g) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
h) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit : J. Organometallic Chemistry., 33, 337 (1971) KOZIMA S.& coll.

On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate.

Il est entendu que les réactions décrites ci-dessus peuvent être effectuées comme indiqué ou encore, le cas échéant, selon d'autres méthodes usuelles connues de l'homme du métier.
i) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.

Le groupement phtalimido peut être éliminé par l'hydrazine.

On trouvera une liste de différents groupements protec-teurs utilisables par exemple dans le brevet BF 2 499 995.
j) Les produits décrits ci-dessus peuvent, si désiré, faire l'objet de réactions de salification par exemple par un acide minéral ou organique ou par une base minérale ou organique selon les méthodes usuelles connues de l'homme du métier.
k) Les éventuelles formes optiquement actives des produits décrits ci-dessus peuvent être préparées par dédoublement des racémiques selon les méthodes usuelles connues de l'homme du métier.

Les éventuelles fonctions réactives qui sont éventuellement protégées sont notamment les fonctions hydroxy ou amino. On utilise pour protéger ces fonctions des groupements protecteurs usuels. On peut citer par exemples les groupements protecteurs suivants du radical amino: tert-butyle, tert-amyle, trichloroacétyle, chloroacétyle, benzhydryle, trityle, formyle, benzyloxycarbonyle.

Comme groupement protecteur du radical hydroxy on peut citer les radicaux tels que formyle, chloroacétyle, tétrahydropyrannyle, triméthyleilyle, tert-butyl diméthylsilyle.

Il est bien entendu que la liste ci-dessus n'est pas limitative et que d'autres groupements protecteurs, par exemple connus dans la chimie des peptides peuvent être utilisés. Une liste de tels groupements protecteurs se trouve par exemple dans le brevet français BF 2.499.995 dont le contenu est incorporé ici par référence.

Les réactions éventuelles d'élimination des groupements protecteurs sont effectuées comme indiqué dans ledit brevet BF 2.499.995. Le mode préféré d'élimination est l'hydrolyse acide à l'aide des acides choisis parmi les acides chlorhydrique, benzène sulfonique ou para toluène sulfonique, formique ou trifluoroacétique. On préfère l'acide chlorhydrique.

La réaction éventuelle d'hydrolyse du groupement >C=NH en groupement cétone est également effectuée de préférence à l'aide d'un acide tel que l'acide chlorhydrique aqueux par exemple au reflux.

Un exemple d'élimination du groupement terbutyldiméthylsilyle au moyen de l'acide chlorhydrique est donné ci-après dans les exemples.
- L'estérification éventuelle d'un radical OH libre est effectuée dans des conditions classiques. On peut utiliser par exemple un acide ou un dérivé fonctionnel, par exemple un anhydride tel que l'anhydride acétique en présence d'une base telle que la pyridine. L'estérification ou la salification éventuelle d'un groupement COOH est effectuée dans les conditions classiques connues de l'homme du métier.

L'amidification éventuelle d'un radical COOH est effectuée dans des conditions classiques. On peut utiliser une amine primaire ou secondaire sur un dérivé fonctionnel de l'acide par exemple un anhydride symétrique ou mixte.

Les produits de départ utilisés pour préparer les produits de formule (Ia) selon la présente invention peuvent être connus et disponibles dans le commerce ou peuvent être préparés selon des méthodes connues de l'homme de métier.

Les produits objet de la présente invention sont doués de propriétés pharmacologiques intéressantes: on a constaté qu'ils possédaient notamment des propriétés inhibitrices de protéines kinases.

Parmi ces protéines kinases, on cite notamment IGF1R.

Des tests donnée dans la partie expérimentale ci-après illustrent l'activité inhibitrice de produits de la présente invention vis-à-vis de telles protéines kinases.

Ces propriétés rendent donc les produits de formule générale (Ia) de la présente invention utilisables comme médicaments pour le traitement de tumeurs malignes.

Les produits de formule (Ia) peuvent également être utilisés dans le domaine vétérinaire.

L'invention a donc pour objet l'application, à titre de médicaments, des produits de formule générale (Ia) tels que définis ci-dessus, lesdits produits de formule (Ia) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (Ia).

Les produits peuvent être administrés par voie parentérals, buccale, perlinguale, rectale ou topique.

L'invention a aussi pour objet les compositions pharmaceutiques, caractérisées en ce qu'elles renferment, à titre de principe actif, un au moins des médicaments de formule générale (Ia).

Ces compositions peuvent être présentées sous forme de solutions ou de suspensions injectables, de comprimés, de comprimés enrobés, de capsules, de sirops, de suppositoire, de crèmes, de pommades et de lotions. Ces formes pharmaceutiques sont préparées selon les méthodes usuelles. Le principe actif peut être incorporé à des excipients habituellement employés dans ces compositions tels que les véhicules aqueux ou non, le talc, la gomme arabique, le lactose, l'amidon, le stéarate de magnésium, le beurre de cacao, les corps gras d'origine animale ou végétale, les dérivés paraffiniques, les glycols, les divers agents mouillants, dispersants ou émulsifiants, les conservateurs.

La dose usuelle, variable selon le sujet traité et l'affection en cause, peut être, par exemple, de 10 mg à 500 mg par jour chez l'home, par voie orale.

La présente invention concerne l'utilisation de produits de formule (Ia) telle que définie ci-dessus ou de sels pharmaceutiquement acceptables desdits produits de formule (Ia) pour la préparation d'un médicament destiné à prévenir ou traiter une maladie appartenant au groupe suivant: désordres de la prolifération de vaisseaux sanguins, désordres fibrotiques, désordres de la prolifération de cellules mésangiales, désordres métaboliques, allergies, asthme, thromboses, malades du système nerveux, rétinopathies, psoriasis, arthrite rhumatoide, diabètes, dégénération musculaire, maladies en oncologie, cancers.

La présent invention concerne particulièrement l'utilisation de produits de formule (Ia) telle que définie ci-dessus ou de sels pharmaceutiquement acceptables desdite produite de formule (Ia) pour la préparation d'un médicament destiné à traiter des cancers.

Parmi ces cancers, la présente invention s'intéresse tout particulièrement au traitement des tumeurs solides ou liquides et au traitement de cancers résistants aux agents cytotoxiques.

Parmi ces cancers, la présente invention concerne tout particulièrement le traitement du cancer du sein, de l'estomac, du colon, des poumons, des ovaires, de l'utérus, du cerveau, du rein, du larynx, du système lymphatique, de la thyroïde, du tractus uro-génital, du tractus incluant vésicule et prostate, du cancer des os, du pancréas, les mélanomes.

La présente invention s'intéresse encore plus particulièrement au traitement du cancer du sein, du colon et des poumons.

La présente invention concerne aussi l'utilisation de produits de formule (Ia) telle que définie ci-dessus ou de sels pharmaceutiquement acceptables desdits produits de formule (Ia) pour la préparation d'un médicament destiné à la chimiothérapie de cancers.

A titre de médicaments selon la présente invention destinés à la chimiothérapie de cancers, les produits de formule (Ia) selon la présente invention peuvent être utilisés seuls ou en association avec chimiothérapie ou radiothérapie ou alternativement en association avec d'autres agents thérapeutiques.

La présente invention concerne ainsi notamment les compositions pharmaceutiques telles que définies ci-dessus contenant en plus, des principes actifs d'autres médicaments de chimiothérapie contre le cancer.

De tels agents thérapeutiques peuvent être des agents anti-tumoraux utilisés communément.

Comme exemples d'inhibiteurs connus de protéines kinases, on peut citer notamment la butyrolactone, le flavopiridol, la 2-(2-hydroxyéthylamino)-6-benzylamino-9-méthylpurine, l'olomucine, le Glivec ainsi que l'Iressa.

Les produits de formule (Ia) selon la présente invention peuvent ainsi également être avantageusement utilisés en combinaison avec des agents anti-prolifératifs : à titre d'exemples de tels agents anti-prolifératifs mais sans toutefois se limiter à cette liste, on peut citer les inhibiteurs d'aromatase, les antiestrogènes, les inhibiteurs de topoisoméraae I, les inhibiteurs de topoisomérase II, les agents actifs sur les microtubules, les agents d'alkylation, les inhibiteurs d'histone désacétylase, les inhibiteurs de farnésyl transférase, les inhibiteurs de COX-2, les inhibiteurs de MMP, les inhibiteurs de mTOR, les antimétabolites antinéoplastique, les composés du platine, les composés faisant décroître l'activité des protéines kinases et également les composés anti-angiogéniques, les agonistes de la gonadoréline, les anti-androgénes) les bengamides, les biphophonates et le trastuzumab.

On peut citer ainsi à titre d'exemples, des agents anti-microtubules comme les taxoides, vinka-alkaloides, des agents d'alkylation tels que cyclophosphamide, des agents DNA-intercalant comme le cis-platinum, des agents interactifs sur topoisomérase comme la camptothécine et dérivés, les anthracyclines comme l'adriamycine, des antimétabolites comme le 5-fluorouracile et dérivées et analogues.

La présente invention concerne donc des produits de formule (Ia) comme inhibiteurs de IGF1R lesdits produits de formule (Ia) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (Ia).

Les spectres de RMN 1H sont enregistrés sur des spectromètres BRUKER à 400 MHz (AVANCE DRX-400) ou à 300 MHz (BRUKER AVANCE DPX-300). Les déplacements chimiques sont donnés en ppm (δ en ppm) - dans le solvant diméthylsulfoxide - d6. (DMSO-d6) référencé à 2,50 ppm à la température de 303K.

Les spectres de Masse ont été réalisés, soit en électrospray (ES) sur appareil Q-Tof-2 (micromans), ZQ (Micromass) et Quattro Premier (Micromass), soit en impact électronique (IB); 70eV; appareil Micromass GCTof Premier, soit en ionisation chimique (IC); gaz réactant ammoniac; appareil Micromass GCTof.

La LCMS est réalisée sur Colonne Hyperail Gold C18 3x50 mm de diamètre particules : 3µm

Conditions initiales :
Solvant A : Eau à 0,05 % TFA 95%
Solvant B : Acétonitrile à 0,05 % TFA 5%
Débit 0,9 mL Pression à t₀ : 145b volume injecté : 5µl GRADIENT sur 7mn

| Temps | %A | %B |
|---|---|---|
| 0 | 95 | 5 |
| 5 | 5 | 95 |
| 5,5 | 5 | 95 |
| 6, 5 | 95 | 5 |
| 7 | 95 | 5 |

Détecteur U.V. DAD : 200<λ<400 nm, la masses est mesurée par électrospray (ES+) sur appareil Q-Tof-2 (Micromass)

Les exemples dont la préparation suit illustrent la présente invention sans toutefois la limiter.

Des exemples de produits ayant différents radicaux NR4R5 selon la présente invention sont indiqués ci-après : ces produits font partie de la présente invention.

### Exemple 1 : 1-(4-{[3-(4-tert-butylphényl)-5,5-diméthyl-2,4-dioxoimidazolidin-1-yl]méthyl}pyridin-2-yl)-3-(3-pyrrolidin-1-ylpropyl)urée

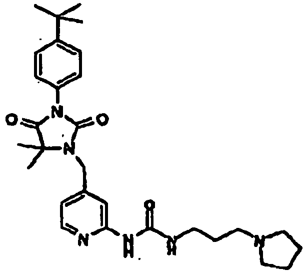

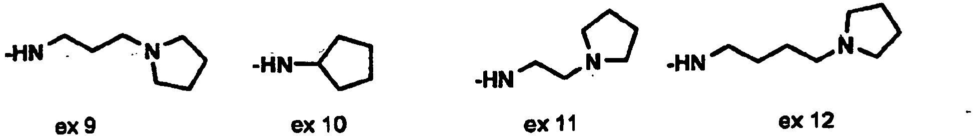

Stade c: 1-(4-{[3-(4-tert-butylphényl)-5,5-diméthyl-2,4-dioxoimidazolidin-1-yl]méthyl}pyridin-2-yl)-3-(3-pyrrolidin-1-ylpropyl)urée A une solution de 100 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenu au stade b) ci-dessous dans 2 mL de diméthylsulfoxyde sont ajoutés 120 mg de 3-pyrrolidin-1-ylpropan-1-amine. La solution est agitée à une température de 100°C durant une heure et quarante minutes. Après retour à une température voisine de 20°C, le milieu réactionnel est dilué par de l'eau, la suspension filtrée et le précipité purifié par chromatographie sur colonne de silice en éluant par un mélange dichlorométhane/méthanol/ammoniaque aqueuse à 28% (gradient de 100/0 à 75/20/5 en volumes). On obtient 18,5 mg de 1-(4-{[3-(4-tert-butylphényl)-5,5-diméthyl-2,4-dioxoimidazolidin-1-yl]méthyl}pyridin-2-yl)-3-(3-pyrrolidin-1-ylpropyl)urée sous forme d'une poudre jaune pâle dont les caractéristiques sont les suivantes :
Spectre de RMN 1H à 400MHz : 1, 31 (s, 9H); 1,40 (s, 6H);
de 1,57 à 1,70 (m, 6H); 2,41 (m, 6H); 3,21 (q, J = 6,5 Hz, 2H); 4,56 (s, 2H); 6,92 (d large, J = 5,5 Hz, 1H); 7,31 (s large, 1H); 7,34 (d large, J = 8,5 Hz, 2H); 7,51 (d large, J = 8,5 Hz, 2H); 8,11 (d, J = 5,5 Hz, 1H); 8,27 (m large, 1H); 9,13 (s, 1H)
Spectre de masse (ES) : m/z=521 [M+H]⁺ (pic de base); m/z=519 [M-H]⁻ (piç de base)

### Stade_b : 3-(4-tert-butylphényl)-5,5-diméthyl-1-[(2-thioxo-2H-[1,2,4]oxadiazolo[2,3-a]pyridin-7-yl)méthyl]imidazolidine-2,4-dione

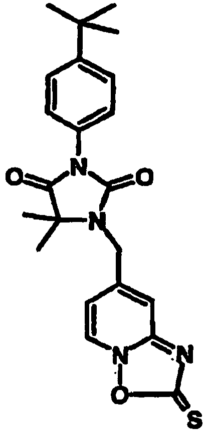

A une solution de 240 mg de N-(4-{[3-(4-tert-butylphényl)-5,5-diméthyl-2,4-dioxoimidazolidin-1-yl]méthyl}-1-oxidopyridin-2-yl)acétamide obtenu au stade a) ci-dessous dans 6 mL d'éthanol sont ajoutés 109 mg d'hydrogànocarbonate de sodium et 75 mg de bichlorure thiocarbonyl. Après deux heures et trente minutes d'agitation, le solide en suspension est filtré, lavé par de l'éthanol et séché. On obtient 220 mg de 3-(4-tert-butylphényl)-5,5-diméthyl-1-[(2-thiooeo-2H-[1,2,4]oxadiazolo[2,3-a]pyridin-7-yl)triéthyl]imidazolidine-2,4-dione sous forme d'une poudre beige don't les caractéristiques sont les suivantes : Spectre de masse (ES) : m/z=425 [M+H]⁺; m/z=423 [M-H]⁻

### Stade a : N-(4-{[3-(4-tert-butylphényl)-5,5-diméthyl-2,4-dioxoimidazolidin-1-yl]méthyl}-1-oxidopyridin-2-yl)acétamide

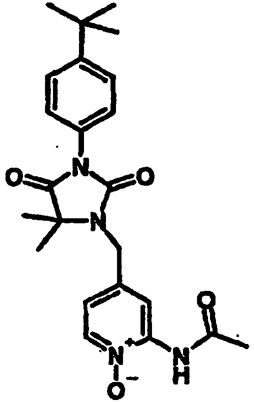

A une solution de 370 mg de N- (4-{[3-(4-tert-butylphényl)-5,5-diméthyl-2,4-dioxoimidazolidin-1-yl]méthyl}pyridin-2-yl)acétamide obtenu au stade z) ci-après dans 20 mL de dichlorométhane sont ajoutés sous argon et sous agitation 704 mg d'acide 3-chlorobenzenecarboperoxyde. Après une heure trente d'agitation à une température voisine de 20°C sont ajoutés 156 mg d'acide 3-chlorobenzenecarboperoxyde. La solution est agitée une nuit puis diluée par du dichlorométhane, lavée par trois fois une solution aqueuse saturée en hydrogénocarbonate de sodium. La phase organique est séchée sur sulfate de magnésium, filtrée et concentrée sous pression réduite. Après chromatographie sur colonne de silice en éluant par un mélange dichlorométhane/méthanol/ammoniaque aqueuse à 28% (gradient de 100/0 à 90/9/1 en volumes), on obtient 280 mg de N-(4-{[3-(4-tert-butylphényl)-5,5-diméthyl-2,4-dioxoimidazolidin-1-yl]méthyl}-1-oxidopyridin-2-yl)acétamide sous forme d'une cire jaune dont les caractéristiques sont les suivantes :
Spectre de masse (ES) : m/z=425 [M+H]⁺; m/z=423 [M-H]⁻

### Stade z : N-(4-{[3-(4-tert-butylphényl)-5,5-diméthyl-2,4-dioxoimidazolidin-1-yl]méthyl}pyridin-2-yl)acétamide

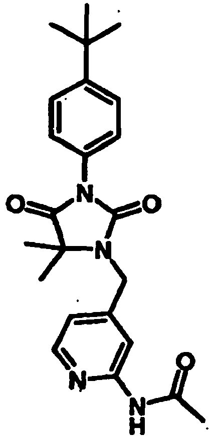

A une solution de 400 mg de 3-(4-tert-butylphényl)-1-[(2-chloropyridin-4-yl)méthyl]-5,5-diméthylimidazolidine-2,4-dione obtenu au stade y ci-après dans 12 mL de dioxanne sont ajoutés successivement sous argon, 23,3 mg de diacétate de palladium, 72 mg de (9,9-diméthyl-9H-xanthène-3,6-diyl)bis(diphénylphosphine)[xantphos], 1,18 g de carbonate de césium et 153 mg d'acétamide. Après une heure de chauffage à une température voisine de 90°C et chromatographie sur colonne de silice en éluant par un mélange d'oxyde de diéthyle/acétate d'éthyle (gradient jusqu'à 100% d'acétate d'éthyle), on obtient 370 mg de N-(4-{[3-(4-tert-butylphényl)-5,5-diméthyl-2,4-dioxoimidazolidin-1-yl]méthyl}pyridin-2-yl)acétamide sous forme de cristaux blanc dont les caractéristiques sont les suivantes :
LCMS : TR = 3,95 min ; m/z=409 [M+H]⁺

### Stade y : 3-(4-tert-butylphényl)-1-[(2-chloropyridin-4-yl)méthyl]-5,5-diméthylimidazolidine-2,4-dione

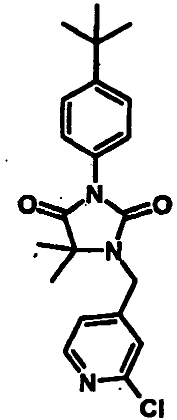

A une suspension de 1,1 g d'hydrure de sodium dans 40 mL de diméthylformamide sous argon sont ajoutés successivement 4,9 g de 3-(4-tert-butylphényl)-5,5-diméthylimidazolidine-2,4-dione obtenu au stade x) ci-après et 4,55 g de 2-chloro-4-(chlorométhyl)pyridine. Le mélange réactionnel est agité pendant 48h à température ambiante puis dilué avec 260 mL d'eau. Le solide formé est filtré, rincé avec de l'ether diisopropylique et séché pour donner 4,61 g de 3-(4-terbutylphényl)-1-[(2-chloropyridin-4-yl)méthyl]-5,5-diméthylimidazolidine-2,4-dione sous forme d'une poudre beige dont les caractéristiques sont les suivantes :
Spectre de masse (ES) : m/z=386 [M+H]⁺; m/z=430 [M-H+HCOOH]-

### stade x) : 3-(4-tert-Butyl-phényl)-5,5-diméthyl-imidazolidine-2,4-dione

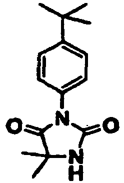

A une suspension de 15 g de 4-tert-butyl-phenylisocyanate dans 200 mL de toluène sont ajoutés successivement 31,52 mL de triéthylamine et 13,15 g de chlorhydrate d'ester méthylique de 2,2-diméthyl-glycine. Le mélange réactionnel est chauffé au reflux pendant 24h, refroidi à température ambiante, versé sur de l'eau distillée et extrait à l'acétate d'éthyle. La phase organique est lavée successivement à l'eau, avec une solution saturée de chlorure de sodium, séchée sur sulfate de magnésium, filtrée et concentrée sous pression réduite. Le résidu est repris dans de l'éther diéthylique et le solide formé est filtré et séché pour donner 17,75 g de 3-(4-tert-butyl-phényl)-5,5-diméthyl-imidazolidine-2,4-dione dont les caractéristiques sont les suivantes :
Spectre RMN 1H à 300 MHz : 1,30 (s, 9H); 1,39 (s, 6H); 7,26 (d, J = 8,5 Hz, 2H), 7,48 (d, J = 8,5 Hz, 2H); 8,48 (s large, 1H).
Spectre de Masse (IE) : m/z=260 M⁺

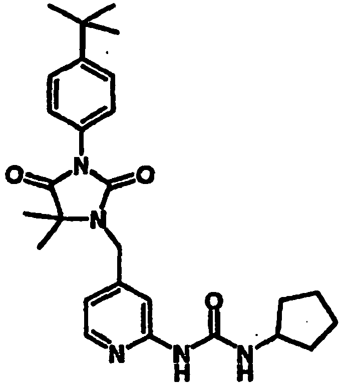

### Exemple 2 : 1-(4-{[3-(4-tert-butylphényl)-5,5-diméthyl-2,4-dioxoimidazolidin-1-yl]methyl}pyridin-2-yl)-3-cyclopentylurée

Peut être préparée comme au stade c) de l'exemple 1 mais à partir de 100 mg de 3-(4-tert-butylphényl)-5,5-diméthyl-1- [ (2-thioxo-2H- [1,2,4] oxadiazolo [2,3-a] pyridin-7-yl)méthyl] imidazolidine-2,4-dione obtenue au stade b) de l'exemple 1, de 2 mL de diméthylsulfoxyde et de 60 mg de cyclopentanamine. Après chromatographie sur colonne de silice en éluant par un mélange d'oxyde de diéthyle/acétate d'éthyle (gradient de 100/0 à 0/100 en volumes), on obtient 33 mg de 1-(4-{ [3- (4-tert-butylphényl)-5,5-diméthyl-2,4-dioxoimidazolidin-1-yl]méthyl}pyridin-2-yl)-3-cyclopentylurée sous forme d'une poudre blanche dont les caractéristiques sont les suivantes :
Spectre de RMN 1H à 400MHz : 1, 31 (s, 9H) ; 1,40 (a, 6H) ; 1,40. (m masqué, 2H) ; 1,56 (m, 2H) ; 1, 65 (m, 2H) ; 1,86 (m, 2H) ; 4,00 (m, 1H) ; 4,56 (s, 2H) ; 6,91 (dd, J = 1,5 et 5,5 Hz, 1H) ; 7,34 (d large, J = 8,5 Hz, 2H) ; 7,36 (s large, 1H) ; 7,51 (d large, J = 8,5 Hz, 2H) ; 8,11 (d, J = 5,5 Hz, 1H) ; 8,18 (m large, 1H) ; 9,09 (s, 1H)
Spectre de masse (ES) : m/z=478 [M+H]⁺ (pic de base) ; m/z=476 [M-H]⁻

### Exemple 3 : 1-(4-{[3-(4-tert-butylphényl)-5,5-diméthyl-2,4-dioxoimidazolidin-1-yl]méthyl}pyridin-2-yl)-3-(2-pyrrolidin-1-yléthyl)urée

Peut être préparée comme au stade c) de l'exemple 1 mais à partir de 100 mg de 3-(4-tert-butylphényl)-5,5-diméthyl-1-[(2-thioxo-2H-[1,2,4]oxadiazolo[2,3-a] pyridin-7-yl)méthyl]imidazolidine-2,4-dione obtenue au stade b) de l'exemple 1, de 2 mL de dioxanne et de 32,3 mg de 2-pyrrolidin-1-yléthanamine. Après chromatographie sur colonne de silice en éluant par un mélange de dichlorométhane/méthanol/ammoniaque aqueuse à 28% (95/4/1 en volumes), on obtient 42,4 mg de 1-(4-{[3-(4-tert-butylphényl)-5,5-diméthyl-2,4-dioxoimidazolidin-1-yl]méthyl}pyridin-2-yl)-3-(2-pyrrolidin-1-yléthyl)urée sous forme d'une poudre blanche dont les caractéristiques sont les suivantes :
Spectre de RMN 1H à 400MHz : 1, 32 (s, 9H) ; 1,40 (s, 6H) ; 1,69 (m, 4H) ; 2,47 (m partiellement masqué, 4H) ; 2,52 (t partiellement masqué, J = J = 6,5 Hz, 2H); 3,27 (q partiellement masqué, J = 6,5 Hz, 2H) ; 4,56 (s, 2H) ; 6,91 (dd, J = 1,5 et 5,5 Hz, 1H) ; 7,34 (d large, J m 9, 0 Hz, 2H) ; 7,35 (s large, 1H) ; 7, 51 (d large, J = 9, 0 Hz, 2H) ; 8,10 (d, J = 5,5 Hz, 1H) ; 8,24 (m large, 1H) ; 9,17 (s large, 1H)
Spectre de masse (ES) : m/z=507 [M+H]⁺

### Exemple 4 :1- (4- {[3- (4-tert-butylphényl) -5,5-diméthyl-2,4-dioxoimidazolidin-1-yl]méthyl}pyridin-2-yl)-3-(4-pyrrolidin-1-ylbutyl)urée

Peut être préparée comme au stade c) de l'exemple 1 mais à partir de 100 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenue au stade b) de l'exemple 1, de 2 mL de dioxanne et de 40,2 mg de 4-pyrrolidin-1-ylbutan-1-amine. Après chromatographie sur colonne de silice en éluant par un mélange de dichlorométhane/méthanol/ammoniaque aqueuse à 28% (95/4/1 en volumes), on obtient 60,2 mg de 1-(4-{[3-(4-tert-butylphényl)-5,5-diméthyl-2,4-dioxoimidazolidin-1-yl]méthyl}pyridin-2-yl)-3-(4-pyrrolidin-1-ylbutyl)urée sous forme d'une poudre blanche dont les caractéristiques sont les suivantes :
Spectre de RMN 1H à 400MHz : 1,32 (s, 9H) ; 1,40 (s, 6H) ; 1,48 (m, 4H) ; 1,65 (m, 4H) ; 2,38 (m, 6H) ; 3,17 (q, J = 6,5 Hz, 2H); 4,56 (s, 2H) ; 6,92 (d large, J = 5,5 Hz, 1H) ; 7,31 (s large, 1H) ; 7,34 (d large, J = 8,5 Hz, 2H) ; 7,51 (d large, J = 8,5 Hz, 2H) ; 8,11 (d, J = 5,5 Hz, 1H) ; 8,25 (m large, 1H) ; 9,12 (s large, 1H)
Spectre de masse (ES) : m/z=535 [M+H]⁺ (pic de base) ; m/z=533 [M-H]⁻

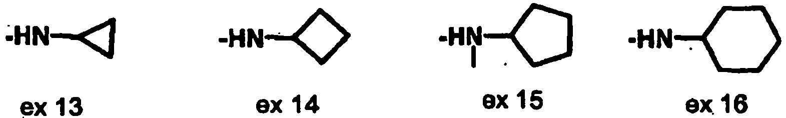

### Exemple 5 :1-(4-([3-(4-tert-butylphényl)-5,5-diméthyl-2,4-dioxoimidazolidin-1-yl] méthyl}pyridin-2-yl)-3-cyclopropylurée

Peut être préparée comme au stade c) de l'exemple 1 mais à partir de 200 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenue au stade b) de l'exemple 1, de 4 mL d'éthanol et de 32,3 mg de cyclopropanamine. Après une heure de chauffage à une température de 50°C, et chromatographie sur colonne de silice en éluant par un mélange d'heptane/acétate d'éthyle (gradient de 100/0 à 0/100 en volumes), on obtient 76,1 mg de 1-(4-([3-(4-tert-butylphényl)-5,5-diméthyl-2,4-dioxoimidazolidin-1-yl]méthyl}pyridin-2-yl)-3-cyclopropylurée sous forme d'une poudre blanche dont les caractéristiques sont les suivantes :
Spectre de RMN 1H à 400MHz : 0,44 (m, 2H) ; 066 (m, 2H) ;1,31 (s, 9H) ; 1,40 (s, 6H) ; 2,60 (m, 1H) ; 4,56 (s, 2H) ; 6,92 (dd, J = 1,5 et 5,5 Hz, 1H) ; 7,34 (d large, J = 8,5 Hz, 2H); 7,36 (s large, 1H) ; 7,52 (d large, J = 8,5 Hz, 2H); 8,11 (d, J = 5,5 Hz, 1H) ; 8,24 (m large, 1H) ; 9,09 (s large, 1H)
   Spectre de masse (ES) : m/z=450 [M+H]⁺ (pic de base)

### Exemple 6 : 1-(4-{[3-(4-tert-butylphényl)-5,5-diméthyl-2,4-dioxoimidazolidin-1-yl]méthyl}pyridin-2-yl)-3-cyclobutylurée

Peut être préparée comme au stade c) de l'exemple 1 mais à partir de 200 mg de 3-(4-tert-butylphényl)-5,5-diméthyl-1-[(2-thioxo-2H-[1,2,4] oxadiazolo[2,3-a]pyridin-7-yl)méthyl]imidazolidine-2,4-dione obtenue au stade b) de l'exemple 1, de 4 mL de dioxanne et de 40,2 mg de cyclobutanamine. Après chromatographie sur colonne de silice en éluant par un mélange d'heptane/acétate d'éthyle (gradient de 100/0 à 0/100 en volumes), on obtient 112,6 mg de 1-(4-{[3-(4-tert-butylphényl)-5,5-diméthyl-2,4-dioxoimidazolidin-1-yl]méthyl}pyridin-2-yl)-3-cyclobutylurée sous forme d'une poudre blanche dont les caractéristiques sont les suivantes :
Spectre de RMN 1H à 400MHz : 1,31 (s, 9H); 1,40 (s, 6H) ; 1,65 (m, 2H) ; 1,88 (m, 2H) ; 2,24 (m, 2H); 4,18 (m, 1H) ; 4,56 (s, 2H) ; 6,92 (dd, J = 1,5 et 5,5 Hz, 1H) ; 7,34 (d large, J = 8,5 Hz, 2H) ; 7,35 (s large, 1H) ; 7,52 (d large, J = 8,5 Hz, 2H) ; 8,13 (d, J = 5,5 Hz, 1H) ; 8,37 (d large, J = 8,0 Hz, 1H) ; 9,07 (s large, 1H)
Spectre de masse (ES) : m/z=464 [M+H]⁺ (pic de base); m/z=462 [M-H]⁻ (pic de base)

### Exemple 7 : 3-(4-{[3-(4-tert-butylphényl)-5,5-diméthyl-2,4-dioxoimidazolidin-1-yl]méthyl}pyridin-2-yl)-1-cyclopentyl-1-méthylurée

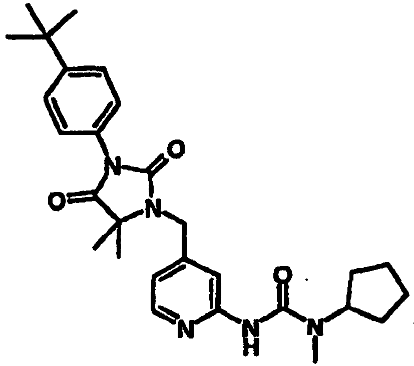

### Sate b) :3-(4-{[3-(4-tert-butylphényl)-5,5-diméthyl-2,4-dioxoimidazolidin-1-yi]méthyl}pyridin-2-yl)-1-cyclopentyl-1-méthylurée

A une solution de 0,16 g (4-{[3-(4-tert-butylphényl)-5,5-diméthyl-2,4-dioxoimidazolidin-1-yl]méthyl}pyridin-2-yl)carbamate d'éthyle obtenu au stade a) ci-dessous dans 2 mL de tétrahydrofuranne sont ajoutés successivement 0,368 mL de N-méthylcyclopentylamine et 0,512 mL de triéthylamine. Le mélange réactionnel est chauffé au microonde à 130°c pendant 3 heures puis concentré sous pression réduite. Le résidu est purifié par chromatogarphie sur colonne de silice en éluant avec un mélange d'acétate d'éthyle et de cyclohexane (60/40 en volumes) pour donner 0,048 g de 3-(4-{[3-(4-tert-butylphényl)-5,5-diméthyl-2,4-dioxoimidazolidin-1-yl]méthyl}pyridin-2-yl)-1-cyclopentyl-1-méthylurée dont les caractéristiques sont les suivantes :
Spectre RMN 1H à 400 MHz : 1,31 (s, 9H); 1,39 (s, 6H) ; de 1,42 à 1,79 (m, 8H) ; 2,82 (s, 3H) ; 4,58 (s, 2H) ; 4,60 (m, 1H) ; 6, 98 (dd, J = 1,5 et 5,5 Hz, 1H) ; 7,33 (d, J = 8, 5 Hz, 2H) ; 7,51 (d, J = 8,5 Hz, 2H); 7,85 (s large, 1H) ; 8,17 (d, J = 5,5 Hz, 1H) ; 8,72 (s, 1H) .
Spectre de masse (ES) : m/z=492 [M+H]⁺

### Stade a) :(4-{[3-(4-tert-butylphényl)-5,5-diméthyl-2,4-dioxoimidazolidin-1-yl]méthyl]pyridin-2-yl)carbamate d'éthyle

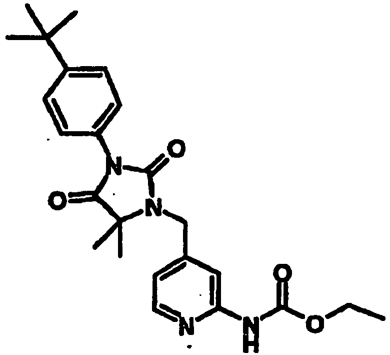

A une solution de 3,5 g de 3-(4-tert-butylphényl)-1-[(2-chloropyridin-4-yl)méthyl]-5,5-diméthylimidazolidine-2,4-dione obtenu au stade a) de l'exemple 1 dans 90 mL de dioxanne sont ajoutés successivement sous argon, 406 mg de diacétate de palladium, 1,1 g de (9,9-diméthyl-9H-xanthène-3,6-diyl)bis(diphénylphosphine) [xantphos] , 12,9 g de carbonate de césium et 1,86 g de carbamate d'éthyle. Le mélange réactionnel eat chauffé à 105°C pendant 7 heures, filtré et concentré sous pression réduite. Le résidu est purifié par chromatographie sur colonne de silice en éluant par un mélange de cyclohéxane et d'acétate d'éthyle (60/40 en volumes) pour donner 1,8 g (4-{[3-(4-tert-butylphényl)-5,5-diméthyl-2,4-dioxoimidazolidin-1-yl]méthyl}pyridin-2-yl)carbamate d'éthyle dont les caractéristiques sont les suivantes :
Spectre RMN 1H à 300 MHz : 1,23 (t, J = 7,5 Hz, 3H) ; 1,31 (s, 9H) ; 1,40 (s, 6H) ; 4,14 (q, J = 7,5 Hz, 2H) ; 4,60 (s large, 2H) ; 7,07 (d large, J = 5,5 Hz, 1H) ; 7,32 (d, J = 8,5 Hz, 2H) ; 7,52 (d, J = 8,5 Hz, 2H) ; 7,86 (s large, 1H) ; 8,20 (d, J = 5,5 Hz, 1H) ; 10,05 (s large, 1H) .
Spectre de masse (ES) : m/z=439 [M+H]⁺; m/z=437 [M+H]⁺

### Exemple 8 : 1-(4-{[3-(4-tert-butylphényl)-5,5-diméthyl-2,4-dioxoimidazolidin-1-yl]méthyl}pyridin-2-yl)-3-cyclohexylurée

Peut être préparée comme au stade c) de l'exemple 1 mais à partir de 200 mg de 3-(4-tert-butylphényl)-5,5-diméthyl-1-[(2-thioxo-2H-[1,2,4]oxadiazolo[2,3-alpyridin-7-yl)méthyl]imidazolidine-2,4-dione obtenue au stade b) de l'exemple 1, de 4 mL de d'éthanol et de 56 mg de cyclohexanamine. Après chromatographie sur colonne de silice en éluant par un mélange d'heptane/acétate d'éthyle (gradient de 100/0 à 0/100 en volumes) , on obtient 92 mg de 1-(4-{[3-(4-tert-butylphényl)-5,5-diméthyl-2,4-dioxoimidazolidin-1-yl]méthyl}pyridin-2-yl)-3-cyclohexylurée sous forme d'une poudre jaune pâle dont les caractéristiques sont les suivantes :
Spectre de RMN 1H à 400MHz : De 1,14 à 1, 44 (m, 5H) ; 1,31 (s, 9H); 1,40 (s, 6H) ; 1,53 (m, 1H) ; 1,66 (m, 2H) ; 1,82 (m, 2H) ; 3,56 (m, 1H) ; 4,56 (s, 2H) ; 6,91 (dd, J = 1,5 et 5,5 Hz, 1H) ; 7,33 (s large, 1H) ; 7,34 (d large, J = 8,5 Hz, 2H) ; 7,51 (d large, J = 8,5 Hz, 2H) ; 8,11 (d, J = 5,5 Hz, 1H) ; 8,20 (m étalé, 1H) ; 9,06 (s, 1H)
Spectre de masse (ES) _{:} m/z=492 [M+H]⁺ (pic de base) m/z=490 [M-H]⁻ (pic de base)

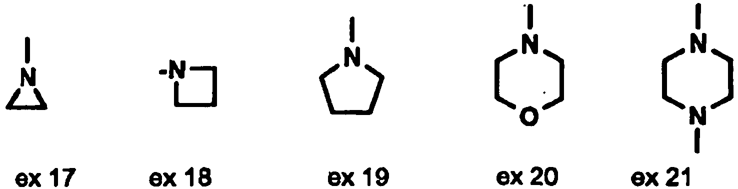

### Exemple 9 : N-(4-{[3-(4-tert-butylphényl)-5,5-diméthyl-2,4-dioxoimidazolidin-1-yl]méthyl]pyridin-2-yl)aziridine-1-carboxamide

### Exemple 10 : N-(4-{[3-(4-tert-butylphényl)-5,5-diméthyl-2,4-dioxoimidazolidin-1-yl]méthyl}pyridin-2-yl)azétidine-1-carboxamide

Peut être préparée comme au stade c) de l'exemple 1 mais à partir de 200 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenue au stade b) de l'exemple 1, de 4 mL de dioxanne et de 32,3 mg d'azétidine. Après quarante cinq minutes de chauffage à une température de 80°C et purification par HPLC (gradient eau-acétonitrile contenant 0,1% d'acide formique), on obtient 29 mg de N-(4-{[3-(4-tert-butylphényl)-5,5-diméthyl-2,4-dioxoimidazolidin-1-yl)méthyl}pyridin-2-yl)azétidine-1-carboxamide sous forme d'une poudre blanche dont les caractéristiques sont les suivantes :
Spectre de RMN 1H à 400MHz : 1,32 (s, 9H) ; 1,40 (s, 6H) ; 2,15 (m, 2H); 3,98 (m, 4H) ; 4,57 (s, 2H); 6,97 (dd, J = 1,5 et 5,5 Hz, 1H) ; 7,33 (d large, J = 8,5 Hz, 2H) ; 7, 51 (d large, J = 8,5 Hz, 2H) ; 7,94 (s large, 1H) ; 8,16 (d, J = 5,5 Hz, 1H) ; 8,95 (s, 1H)
Spectre de masse (ES) _{:} m/z=450 [M+H]⁺ (pic de base)

### Exemple 11 : N-(4-{[3-(4-tert-butylphényl)-5,5-diméthyl-2,4-dioxomidazolidin-1-yl]méthyl}pyridin-2-yl)pyrrolidine-1-carboxamide

Peut être préparée comme au stade c) de l'exemple 1 mais à partir de 200 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenue au stade b) de l'exemple 1, de 4 mL de dioxanne et de 40,2 mg de pyrrolidine. Après chromatographie sur colonne de silice en éluant par un mélange d'heptane/acétate d'éthyle (gradient de 100/0 à 0/100 en volumes), on obtient 64,3 mg de N- (.4- {[3-(4-tert-butylphényl)-5,5-diméthyl-2,4-dioxoimidazolidin-1-yl]méthyl}pyridin-2-yl)pyrrolidine-1-carboxamide sous forme d'une poudre beige dont les caractéristiques sont les suivantes :
Spectre de RMN 1H à 400MHz : 1,31 (s, 9H); 1,40 (s, 6H) ; 1,84 (m, 4H) ; 3,40 (m, 4H) ; 4, 58 (s, 2H) ; 6,98 (dd, J = 1,5 et 5, 5 Hz, 1H) ; 7,33 (d, J = 9,0 Hz, 2H) ; 7,51 (d, J **=** 9,0 Hz, 2H) ; 7,93 (s large, 1H) ; 8,16 (d, J = 5,5 Hz, 1H) ; 8,59 (s, 1H)
Spectre de masse (ES) : m/z=464 [M+H]⁺ (pic de base)

### Exemple 12 :N-(4-{[3-(4-tert-butylphényl)-5,5-diméthyl-2,4-dioxoimidazolidin-1-yl]méthyl}pyridin-2-yl)morpholine-4-carboxamide

Peut être préparée comme au stade c) de l'exemple 1, mais à partir de 150 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenue au stade b) de l'exemple 1, de 3 mL de dioxanne et de 36, 9 mg de morpholine. Après chromatographie sur colonne de silice en éluant par un mélange d'heptane/acétate d'éthyle (gradient de 100/0 à 0/100 en volumes) , on obtient 74 mg de N-(4-([3-(4-tert-butylphényl)-5,5-diméthyl-2,4-dioxoimidazolidin-1-yl]méthyl}pyridin-2-yl)morpholine-4-carboxamide sous forme d'une cire jaune dont les caractéristiques sont les suivantes :
Spectre de RMN 1H à 400MHz : 1,32 (s, 9H) ; 1,39 (s, 6H) ; 3,45 (m, 4H) ; 3,59 (m, 4H) ; 4,58 (s, 2H) ; 7,00 (dd, J = 1,5 et 5,5 Hz, 1H) ; 7,33 (d large, J = 8, 5 Hz, 2H) ; 7,51 (d large, J = 8,5 Hz, 2H) ; 7,84 (s large, 1H) ; 8,18 (d, J = 5,5 Hz, 1H) ; 9,17 (s, 1H)
Spectre de masse (ES) _{:} m/z=480 [M+H]⁺ (pic de base)

### Exemple 13 : (4-{[3-(4-tert-butylphényl)-5,5-diméthyl-2,4-dioxoimidazolidin-1-yl]méthyl}pyridin-2-yl)-4-méthylpipérazine-1-carboxamide

Une solution de 100 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenu au stade b) de l'exemple 1 dans 2 mL de dioxanne et de 31 µL de N-méthylpipérazine est chauffée au microonde à 130°C pendant 15 minutes. Le mélange réactionnel est concentré sous pression réduite et le résidu purifié par HPLC (gradient eau-acétonitrile contenant 0,1% d'acide formique) pour donner 94 mg de N-(4-{[3-(4-tert-butylphényl)-5,5-diméthyl-2,4-dioxoimidazolidin-1-yl]méthyl}pyridin-2-yl)-4-méthylpipérazine-1-carboxamide dont les caractéristiques sont les suivantes :
Spectre RMN 1H à 400 MHz : 1,30 (s, 9H) ; 1,40 (s, 6H); 2,19 (s, 3H) ; 2,29 (m, 4H) ; 3,46 (m, 4H); 4,58 (s, 2H) ; 6,99 (d large, J = 5,5 Hz, 1H) ; 7,33 (d, J = 8,5 Hz, 2H) ; 7,51 (d, J = 8,5 Hz, 2H) ; 7,81 (s large, 1H) ; 8,18 (d, J = 5,5 Hz, 1H) ; 9,13 (s large, 1H).
Spectre de Masse (ES) _{:} m/z=493 [M+H]⁺
   m/z=267,6 [M + CH₃CN + H]²⁺ / 2 pic de base m/z=247 [M + 2H]²⁺ / 2

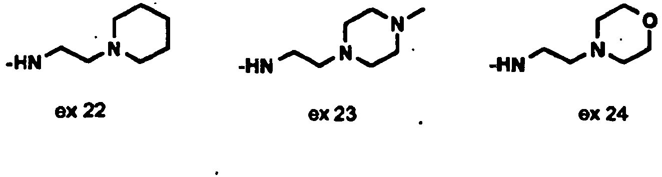

### Exemple 14 :1- (4-{[3-(4-tert-butylphényl)-5,5-diméthyl-2,4-dioxoimidazolidin-1-yl]méthyl}pyridin-2-yl)-3-(2-pipéridin-1-yléthyl)urée

Une solution de 100 mg de 3-4-tert-butylphényl)-5,5-diméthyl-1- [(2-thioxo-2H-[1,2,4] oxadiazolo [2,3-a]pyridin-7-yl)méthyl]imidazolidine-2,4-dione obtenu au stade b) de l'exemple 1 dans 2 mL de dioxanne et de 36 µL de 1- (2-aminoéthyl)pipéridine est chauffée au microonde à 130°C pendant 15 minutes. Le mélange réactionnel est concentré sous pression réduite et le résidu purifié par HPLC (gradient eau-acétonitrile contenant 0,1% d'acide formique) pour donner 75 mg de 1-(4-{[3-(4-tert-butylphényl)-5,5-diméthyl-2,4-dioxoimidazolidin-1-yl]méthyl}pyridin-2-yl)-3-(2-pipéridin-1-yléthyl)urée dont les caractéristiques sont les suivantes : Spectre RMN 1H à 400 MHz : 1,31 (s, 9H); 1,40 (m, 8H); 1,52 (m, 4H); 2,44 (m, 6H); 3,27 (q, J = 6,0 Hz, 2H); 4,56 (s, 2H); 6,91 (d large, J = 5,5 Hz, 1H); 7,29 (s large, 1H); 7,33 (d, J = 8,5 Hz, 2H); 7,51 (d, J = 8,5 Hz, 2H); 8,11 (d, J = 5,5 Hz, 1H); 8,43 (m large, 1H); 9,23 (s large, 1H).
Spectre de Masse (ES) : m/z=521 [M+H]⁺; m/z=281 [M + CH₃CN + H)²⁺ / 2; m/z=261 [M + 2H)²⁺ / 2 pic de base

### Exemple 15 1-(4-{[3-(4-tert-butylphényl)-5,5-diméthyl-2,4-dioxoimidazolidin-1-yl]méthyl}pyridin-2-yl)-3-[2-(4-méthylpipérazin-1-yl)éthyl]urée

Une solution de 100 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenu au stade b) de l'exemple 1 dans 2 mL de dioxanne et de 48 µL de 1-(2-aminoéthyl)-4-méthylpipérazine est chauffée au microonde à 130°C pendant 15 minutes. Le mélange réactionnel est concentré sous pression réduite et le résidu purifié par chromatographie sur colonne de silice en éluant par un gradient (100/0 à 75/25 en volumes) de dichlorométhane et de méthanol/ammoniaque (6/1 en volumes) pour donner 75 mg de 1-(4-{[3-(4-tert-butylphényl)-5,5-diméthyl-2,4-dioxoimidazolidin-1-yl]méthyl}pyridin-2-yl)-3-[2-(4-méthylpipérazin-1-yl)éthyl]urée dont les caractéristiques sont les suivantes :
Spectre RMN 1H à 400 MHz : 1,31 (s, 9H); 1,40 (s, 6H); 2,15 (s, 3H); de 2,25 à 2,45 (m, 10H); de 3,35 à 3,45 (m masqué, 2H); 4,55 (s, 2H); 6,92 (d large, J = 5,5 Hz, 1H); 7,28 (s large, 1H); 7,34 (d, J = 8,5 Hz, 2H); 7,52 (d, J = 8,5 Hz, 2H); 8,11 (d, J = 5,5 Hz, 1H); 8,47 (t large, J = 6,0 Hz, 1H); 9,22 (s large, 1H) .
Spectre de Masse (ES) _{:} m/z=536 [M+H]⁺; m/z=480 [M - tBu + 2H]⁺ pic de base

### Exemple 16 : 1-(4-{[3-(4-tert-butylphényl)-5,5-diméthyl-2,4-dioxoimidazolidin-1-yl]méthyl}pyridin-2-yl)-3-(2-morpholin-4-yléthyl)urée

Une solution de 100 mg de 3-(4-tert-butylphényl)-5,5-diméthyl-1-[(2-thioxo-2H-[1,2,4]oxadiazolo(2,3-alpyridin-7-yl)méthyl]imidazolidine-2,4-dione obtenu au stade b) de l'exemple 1 dans 2 mL de dioxanne et de 37 µL de 1- (2-aminoéthyl)morpholine est chauffée au microonde à 130°C pendant 15 minutes. Le mélange réactionnel est concentré sous pression réduite et le résidu purifié par chromatographie sur colonne de silice en éluant par un gradient (100/0 à 75/25 en volumes) de dichlorométhane et de méthanol/amtnoniaque (6/1 en volumes) pour donner 77 mg de 1-(4-{[3-(4-tert-butylphényl)-5,5-diméthyl-2,4-dioxoimidazolidin-1-yl]méthyl}pyridin-2-yl)-3-(2-morpholin-4-yléthyl)urée dont les caractéristiques sont les suivants:
Spectre RMN 1H à 400 MHz : 1,31 (s, 9H), 1,40 (s, 6H); 2,40 (m, 6H); de 3,35 à 3,45 (m masqué, 2H); 3,60 (m, 4H), 4,56 (s, 2H); 6,93 (d large, J = 5, 5 Hz, 1H); 7,29 (s large, 1H), 7,34 (d, J = 8,5 Hz, 2H); 7,52 (d, J = 8,5 Hz, 2H); 8,12 (d, J **=** 5,5 Hz, 1H); 8,48 (m large, 1H); 9,22 (s large, 1H) .
Spectre de Masse (ES) : m/z=523 [M+H]⁺
   m/z=282,6 [M + CH₃CN + H]²⁺ / 2 pic dé base
   m/z=262 [M + 2H]²⁺ / 2

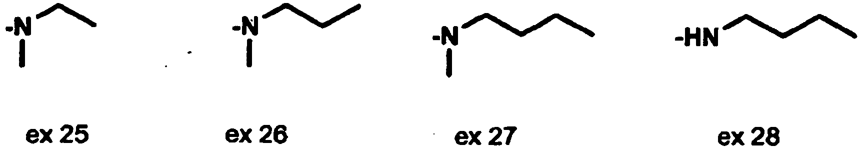

### ex 25 ex 26 ex 27 ex 28 Exemple 17 : 3-(4-{[3-(4-tert-butylphényl)-5,5-diméthyl-2,4-dioxoimidazolidin-1-yl]méthyl}pyridin-2-yl)-1-éthyl-1-mathylurée

Une solution de 100 mg de 3-(4-tert-butylphényl)-5,5-diméthyl-1-[(2-thioxo-2H-[1,2,4]oxadiazolo[2,3-alpyridin-7-yl)méthyl]imidazolidine-2,4-dione obtenu au stade b) de l'exemple 1 dans 2 mL de dioxanne et de 24 µL de N-éthyl-N-méthylamine est chauffée au microonde à 130°C pendant 15 minutes. Le mélange réactionnel est concentré sous pression réduite et le résidu purifié par chromatographie sur colonne de silice en éluant par un mélange d'éther de pétrole et d'acétate d'éthyle (gradient 40/60 à 0/100 en volumes) puis par HPLC (gradient eau-acétonitrile contenant 0,1% d'acide formique) pour donner 12 mg 3-(4-{[3-(4-tert-butylphényl)-5,5-diméthyl-2,4-dioxoimidazolidin-1-yl]méthyl}pyridin-2-yl)-1-éthyl-1-méthylurée dont les caractéristiques sont les suivantes:
Spectre RMN 1H à 400 MHz : 1,07 (t, J = 7,0 Hz, 3H); 1,31 (s, 9H); 1,40 (s, 6H); 2,93 (s, 3H); de 3,35 à 3,45 (m masqué, 2H); 4,59 (s, 2H); 6,99 (d large, J = 5,5 Hz, 1H); 7,33 (d, J = 8,5 Hz, 2H) ; 7,51 (d, J = 8,5 Hz, 2H); 7,86 (s large, 1H); 8,18 (d, J = 5,5 Hz, 1H); 8,71 (s large, 1H).
Spectre de Masse (ES) : m/z=452 [M+H]⁺ pic de base

### Exemple 18 : 3-(4-{[3-(4-tert-butylphényl)-5,5-diméthyl-2,4-dioxoimidazolidin-1-yl]méthyl}pyridin-2-yl)-1-méthyl-1-propylurée

Une solution de 100 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenu au stade b) de l'exemple 1 dans 2 mL de dioxanne et de 29 µL de N-méthyl-N-propylamine est chauffée au microonde à 130°C pendant 15 minutes. Le mélange réactionnel est concentré sous pression réduite et le résidu purifié par chromatographie sur colonne de silice en éluant par un mélange d'éther de pétrole et d'acétate d'éthyle (40/60 en volumes) pour donner 61 mg de 3-(4-{[3-(4-tert-butylphényl)-5,5-diméthyl-2,4-dioxoimidazolidin-1-yl]méthyl}pyridin-2-yl)-1-méthyl-1-propylurée sous forme d'une poudre blanche dont les caractéristiques sont les suivantes :
Spectre RMN 1H à 400 MHz : 0,85 (t, J = 7,5 Hz, 3H); 1, 31 (s, 9H); 1,39 (s, 6H); 1,52 (m, 2H); 2,94 (s, 3H); de 3,25 à 3,35 (m manqué, 2H); 4,58 (s, 2H); 6,98 (dd, J = 1,5 et 5,5 Hz, 1H); 7,33 (d, J = 8,5 Hz, 2H); 7,50 (d, J = 8,5 Hz, 2H); 7,85 (s large, 1H); 8,18 (d, J = 5,5 Hz, 1H); 8,72 (s large, 1H).
ES m/z=466 [M+H]⁺ pic de base

### Exemple 19 :1-butyl-3-(4-{[3-(4-tert-butylphényl)-5,5-diméthyl-2,4-dioxoimidazolidin-1-yl]méthyl}pyridin-2-yl)-1-mêthyluré

Peut être préparée comme au stade c) de l'exemple 1 mais à partir de 150 mg de 3-(4-tert-butylphényl)-5,5-diméthyl-1-[(2-thioxo-2H-[1,2,4]oxadiazolo[2,3-alpyridin-7-yl)méthyl]imidazolidine-2,4-dione obtenue au stade b) de l'exemple 1 de 3 mL de dioxanne et de 36,9 mg de N-méthylbutan-1-amine. Après chromatographie sur colonne de silice en éluant par un mélange d'heptane/acétate d'éthyle (gradient de 100/0 à 0/100 en volumes), on obtient 77,8 mg de 1-butyl-3-(4-{[3-(4-tert-butylphényl)-5,5-dimethyl-2,4-dioxoimidazolidin-1-yl]méthyl}pyridin-2-yl)-1-méthylurée sous forme d'une poudre blanche dont les caractéristiques sont les suivantes :
Spectre de RMN 1H à 400MHz : 0,90 (t, J = 7,5 Hz, 3H); 1,26 (m, 2H); 1,31 (s, 9H); 1,39 (s, 6H); 1,48 (m, 2H); 2,95 (s, 3H); 3,32 (t partiellement masqué, J = 7,5 Hz, 2H); 4,58 (s, 2H); 6, 98 (dd, J = 1,5 et 5,5 Hz, 1H); 7,34 (d large, J = 8,5 Hz, 2H); 7,51 (d large, J = 8,5 Hz, 2H); 7,85 (s large, 1H); 8,17 (d, J =. 5,5 Hz, 1H); 8,70 (s, 1H)
Spectre de masse (ES) : m/z=480 [M+H]⁺ (pic de base)

### Example 20 : 1-butyl-3-(4-{[3-(4-tert-butylphényl)-5,5-diméthyl-2,4-dioxoimidazolidin-1-yl]méthyl}pyridin-2-yl) urée

Peut être préparée comme au stade c) de l'exemple 1 mais à partir de 200 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenue au stade b) de l'exemple 1 de 4 mL de dioxanne et de 41,3 mg de butan-1-amine. Après chromatographie sur colonne de silice en éluant par un mélange d'heptane/acétate d'éthyle (gradient de 100/0 à 0/100 en volumes), on obtient 150,7 mg de 1-butyl-3-(4-{(3-(4-tert-butylphényl)-5,5-diméthyl-2,4-dioxoimidazolidin-1-yl]méthyl}pyridin-2-yl)urée sous forme d'une poudre blanche dont les caractéristiques sont les suivantes :
Spectre de RMN 1H à 400MHz : 0,90 (t, J = 7,5 Hz, 3H); 1,31 (s, 9H); 1,33 (m, 2H); 1,40 (s, 6H); 1,45 (m, 2H); 3,17 (q, J = 7,0 Hz, 2H); 4,56 (s, 2H); 6,91 (dd, J = 1,5 et 5,5 Hz, 1H); 7,31 (s large, 1H); 7,34 (d large, J = 8,5 Hz, 2H); 7,52 (d large, J = 8,5 Hz, 2H); 8,11 (d, J = 5,5 Hz, 1H); 8,24 (m large; 1H); 9,12 (s, 1H)
Spectre de masse (ES) : m/z=466 [M+H]⁺ (pic de base)

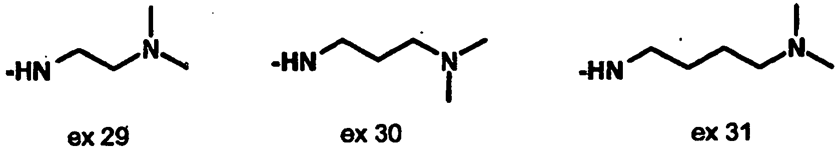

### Exemple 21 : 1-(4-{[3-(4-tert-butylphényl)-5,5-diméthyl-2,4-dioxoimidazolidin-1-yl]méthyl}pyridin-2-yl)-3-[2-(diméthylamino)éthyl]urée

Une solution de 100 mg de 3-(4-tert-butylphényl)-5,5-diméthyl-1-[(2-thioxo-2H-[1,2,oxadiazolo[2,3-a]pyridin-7-yl)méthyl]imidazolidine-2,4-dione obtenu au stade b) de l'exemple 1 dans 2 mL de dioxanne et de 35 µL de N,N-diméthyl-1,3-éthylènediamine est chauffée au microonde à 130°C pendant 15 minutes. Le mélange réactionnel est concentré sous pression réduite et le résidu purifié par chromatographie sur colonne de silice en éluant par un gradient (100/0 à 75/25 en volumes) de dichlorométhane et de méthanol/ammoniaque (6/1 en volume) pour donner 72 mg de 1- (4-{[3-(4-tert-butylphényl) -5,5-diméthyl-2,4-dioxoimidazolidin-1-yl]méthyl}pyridin-2-yl)-3-(2-(diméthylamino)éthyl]urée dont les caractéristiques sont les suivantes :
Spectre RMN 1H à 400 MHz : 1, 31 (s, 9H) ; 1, 40 (s, 6H); 2,19 (s, 6H); 2,38 (m, 2H); 3,35 (q, J = 6,0 Hz, 2H); 4,56 (s, 2H); 6,91 (dd, J = 1,5 et 5,5 Hz, 1H); 7,34 (m, 3H); 7,51 (d, J = 8,5 Hz, 2H); 8,11 (d, J = 5,5 Hz, 1H); 8,19 (m large, 1H); 9,19 (s large, 1H) .
Spectre de Masse (ES) : m/z=481 [M+H]⁺ pic de base

### Exemple 22 : 1-(4-{[3-(4-tert-butylphényl)-5,5-diméthyl-2,4-dioxoimidazolidin-1-yl]méthyl}pyridin-2-yl)-3-[3-(diméthylamino)propyl]urée

Une solution de 100 mg de 3-(4-tert-butylphényl)-5,5-diméthyl-1-[(2-thioxo-2H-[1,2,4] oxadiazolo[2,3-a]pyridin-7-yl)méthyl]imidazolidine-2,4-dione obtenu au stade b) de l'exemple 1 dans 2 mL de dioxanne et de 35 µL de N,N-diméthyl-1,3-propanediamine est chauffée au microonde à 130°C pendant 15 minutes. Le mélange réactionnel est concentré sous pression réduite et le résidu purifié par chromatographie sur colonne de silice en éluant par un mélange de dichlorométhane/méthanol/ammoniaque (75/23/2 en volumes) pour donner 72 mg de 1-(4-{ [3-(4-tert-butylphényl)-5,5-diméthyl-2,4-dioxoimidazolidin-1-yl]méthyl}pyridin-2-yl)-3-[3-(diméthylamino)propyl]urée dont les caractéristiques sont les suivantes :
Spectre RMN 1H à 400 MHz : 1,31 (s, 9H) ; 1,40 (s, 6H); 1,60 (m, 2H); 2,16 (s, 6H); 2,29 (m, 2H); 3,19 (q, J = 6,0 Hz, 2H); 4,57 (s, 2H); 6,91 (d large, J = 5,5 Hz, 1H); 7,29 (s large, 1H); 7,33 (d, J = 8,5 Hz, 2H); 7,51 (d, J = 8,5 Hz, 2H); 8,11 (d, J = 5,5 Hz, 1H); 8,33 (m large, 1H); 9,17 (s large, 1H) .
Spectre de Masse (ES) : m/z=495 [M+H]⁺ pic de base

### Exemple 23 : 1-(4-{[3-(4-tert-butylphényl)-5,5-diméthyl-2,4-dioxoimidazolidin-1-yl]méthyl}pyridin-2-yl)-3-[4-(diméthylamino)butyl]urée

Une solution de 100 mg de 3-(4-tert-butylphényl)-5,5-diméthyl-1-[(2-thioxo-2H-[1,2,4]oxadiazolo[2,3-a]pyridin-7-yl)méthyl]imidazolidine-2,4-dione obtenu au stade b) de l'exemple 1 dans 2 mL de dioxanne et de 35 µL de N,N-diméthyl-1,3-propanediamine est chauffée au microonde à 130°C pendant 15 minutes. Le mélange réactionnel est concentré sous pression réduite et le résidu purifié par chromatographie sur colonne de silice en éluant par un mélange de dichlorométhane/méthanol/ammoniaque (75/23/2 en volumes) pour donner 77 mg de 1-(4-{[3-(4-tert-butylphényl)-5,5-diméthyl-2,4-dioxoimidazolidin-1-yl]méthyl}pyridin-2-yl)-3-[4-(diméthylamino)butyl]urêe dont les caractéristiques sont les suivantes :
Spectre RMN 1H à 400 MHz : 1,31 (s, 9H); 1,40 (s, 6H) ; 1,45 (m, 4H); 2,13 (s, 6H); 2,25 (m, 2H); 3,17 (q, J = 6,0 Hz, 2H); 4,56 (s, 2H); 6,91 (dd, J = 1,5 et 5,5 Hz, 1H); 7,30 (s large, 1H); 7,34 (d, J = 8,5 Hz, 2H); 7,52 (d, J = 8,5 Hz, 2H); 8,11 (d, J = 5,5 Hz, 1H); 8,28 (t large, J = 6,0 Hz, 1H); 9,14 (s large, 1H)
Spectre de Masse (ES) : m/z=509 [M+H]⁺ pic de base

### Tests biologiques in vitro

### A) Protocole expérimental pour le test kinase IGF-1R :

L'activité inhibitrice des composés sur IGF1R est déterminée par une mesure de l'inhibition de l'autophosphorylation de l'enzyme en utilisant un test de fluorescence résolue dans le temps (HTRF). Le domaine cytoplasmique humain d'IGF-1R a été cloné en fusion avec la glutathione S-transferase (GST) dans le vecteur d'expression baculovirus pFastBac-GST. La protéine est exprimée dans les cellules SF21 et purifiée à environ 80% d'homogénéité. Pour le test enzymatique, le composé à tester 10 mM en solution dans le DMSO est dilué par étapes 1/3 dans un tampon 50mM Hepes, pH 7.5, 5mM MnCl₂, 50mM NaCl, 3% Glycerol, 0.025% Tween 20, Pour la mesure de l'inhibition, les dilutions successives du composé sont préincubées 30 min et 90 min en présence de 5 nM d'enzyme, la concentration finale de DMSO n'excédant pas 1%. La réaction enzymatique est initiée pour avoir 120 µM d'ATP final et stoppée après 5 min par addition de tampon 100 mM Hepes, pH 7.0, contenant 0.4 M de fluorure de potassium, 133 mM EDTA, BSA 0.1%, l'anticorps anti-GST marqué avec XL665 et l' anticorps anti-phosphotyrosine conjugé au cryptate d'europium Eu-K (Cis-Bio Int.). Les caractéristiques des deux fluorophores, XL-665 and Eu-K, sont disponibles dans G. Mathis et al., Anticancer Research, 1997, 17, pages 3011-3014. Le transfert d'énergie entre le cryptate d'europium excité vers le XL665 accepteur est proportionnel au degré d'autophosphorylation de IGF-1R. Le signal de longue durée spécifique de XL-665 est mesuré dans un compteur de plaques GENios Pro TECAN. L'inhibition de l'autophosphorylation de IGF-1R au temps 30 min et 90 min avec les composés testés de l'invention sont calculés par rapport à un contrôle 1%. DMSO, dont l'activité est mesurée en l'absence de composé. Une courbe représentant le % d'inhibition en fonction du log de la concentration est établie pour déterminer la concentration correspondante à 50% d'inhibition (IC₅₀).

### B) Mesure de l' autophosphorylation d'IGF-1R dans les cellules MCF7 après stimulation par IGF-1

culture cellulaire et réalisation de l'essai : L'autophosphorylation d'IGF1R dans les cellules induites par IGF1 est évaluée par une technique d'ELISA (Enzyme Linked ImmunoSorbent Assay). Les cellules MCF-7 sont ensemencées à 60,000 cellules par puits dans des plaques 6 puits et incubées à 37°C, 5% CO₂ dans du milieu contenant 10% de serum. Après une nuit en 10% sérum, les cellules sont déprivées en sérum pendant 24 heures. Les composés sont ajoutés au milieu 1 heure avant la stimulation par IGF1. Après 10 minutes de stimulation par IGF1, les cellules sont lysées avec un tampon (Hepes 50 mM pH 7.6, Triton X100 1%, Orthovanadate 2 mM, coktail d'inhibiteurs de protéases). Les lysats cellulaires sont incubés sur une plaque 96-puits pre-coatée avec un anticorps anti-IGP1R, suivie d'une incubation avec un anticorps auti-phosphotyrosine couplé à l'enzyme peroxydase. Le niveau de l'activité peroxydase (measuré par DO avec un substrat luminescent) reflète le status de phosphorylation du recepteur.

Calcul des résultats :
(i) Les essais sont effectués en double exemplaire et la moyenne des deux essais est calculée.
(ii) La valeur du signal de la réponse maximum est calculée à partir du contrôl positif : cellules stimulées par IGF1 sans composé.
(iii) La valeur du signal de la réponse minimum est calculée à partir du contrôl négatif: cellules nonstimulées par IGF1 sans composé.
(iv) En utilisant ces valeurs comme maximum (100%) et minimum (0%) respectivement, les données ont été normalisées de façon à donner un pourcentage de la réponse maximum.
(v) Une courbe de dose réponse est tracée et l'IC₅₀ (la concentration à laquelle le composé induit une diminution de 50% du signal) du composé est calculée par analyse en regression non-inéaire.

### C) Mesure de la prolifération/viabilité des MEF-IGF1R

Culture cellulaire : les cellules MEF-IGF1R (clone stable de cellules transfectées par le récepteur HIGF-IR) sont mises en culture à 37°C sous 5% de CO2 dans un milieu EMEM contenant 10% de SVF.

Procédure du test: Les cellules sont ensemencées à 5,000 cellules par puits dans des plaques Cytostar 96 puits (Amersham) avec 0.2 mL de milieu de culture EMEM à 37°C pendant 18 heures. Les cellules sont ensuite lavées deux fois avec du milieu EMEM et laissées en culture sans sérum pendant 24 heures. Les composés sont ensuite ajoutés à différentes concentrations en présence de rhIGF1 (100ng/ mL) et 0.1µCi de Thymidine [¹⁴C] (activité spécifique - 50 mCi/mmol) pour donner 0.2 mL de volume par puits. Après une incubation de 72 heures en présence du composé, à 37°C sous 5% CO₂ l'incorporation de Thymidine [¹⁴C] est mesurée par comptage de la radioactivité sur un compteur Microbeta trilux (Perkin-elmer). La détermination de l'IC50 est réalisée à partir de 10 concentrations croissantes du composé.

Calcul des résultats :
(i) Les essais sont effectués en double exemplaire et la moyenne des deux essais est calculée.
(ii) La valeur du signal de la réponse maximum est calculée à partir du contrôle positif : cellules stimulées par IGF1 sans composé.
(iii) La valeur du signal de la réponse minimum est calculée à partir du contrôle négatif: cellules non stimulées par IGFI sans composé.
(iv) En utilisant ces valeurs comme maximum (100%) et minimum (0%) respectivement, les données ont été normalisées de façon à donner un pourcentage de la réponse maximum.
(v) Une courbe de dose réponse est tracée et l'IC₅₀ (la concentration à laquelle le composé induit une diminution de 50% du signal) du composé est calculée par analyse en régression non-linéaire.

Le tableau suivant donne les activités de certains exemples de la présente invention dans les 3 tests A, B et C décrits ci-dessus :

| Exemples | Test A* | | Test B * | Test C * |
|---|---|---|---|---|
| | 30ʹ | 90ʹ | | |
| Exemple 1 | ++ | ++ | | + |
| Exemple 2 | ++ | ++ | | + |
| Exemple 3 | ++ | ++ | | + |
| Exemple 4 | ++ | +++ | | + |
| Exemple 5 | ++ | ++ | ++ | ++ |
| Exemples 6 | ++ | ++ | ++ | ++ |
| Exemple 8 | + | ++ | | + |
| Exemple 10 | ++ | ++ | +++ | ++ |
| Exemple 11 | ++ | ++ | +++ | ++ |
| Exemple 12 | ++ | +++ | | + |
| Exemple 19 | ++ | ++ | | + |
| Exemple 20 | ++ | ++ | ++ | + |

| | | | | |
|---|---|---|---|---|
| * Pour les tests A, B et C les IC₅₀ (nM) sont répartis comme suit : + > 100nM 10nM < ++ < 100 nM +++ < 10 nM | | | | |

Les produits de formule (Ia) telle que définie ci-deesus dans lesquels le radical NR4R5 a les valeurs indiquées ci-dessus numérotées en ex 1 à ex 23 correspondent respectivement aux exemples 9 à 31 qui appartiennent à la présente invention : les produits des exemples 1 à 23 sont préparée comme indiqué dans les schémas généraux de synthèse décrits ci-dessus.

Une méthode générale de préparation des urées, pour la préparation des produits des exemples 1 à 23 est la suivante : on mélange 0.3 mmole de {4-[3-(4-tert-Butyl-phenyl)-5,5-dimethyl-2,4-diozo-imidazolidin-1-ylmethyl]-pyridin-2-yl}-carbamate d'éthyle et 3 mole de l'amine appropriée et on chauffe dans 3 mL de N-méthyl pyrrolidinone pendant 2 heures au microonde à 130°C et on obtient ainsi l'urée correspondante attendue.

Le {4-[3-(4-tert-Butyl-phenyl)-5,5-dimethyl-2,4-dioxo-imidazolidin-1-ylmethyl]-pyridin-2-yl}-carbamate'd'ethyle peut être préparé à partir du 3-(4-tert-Butyl-phenyl)"-1-(2-chloro-pyridin-4-ylmethyl)-5,5-dimethyl-imidazolidine-2,4-dione et de carbamate d'éthyle par couplage au palladium comme décrit dans le Schéma Général décrit ci-dessus.

Le 3-(4-tert-Butyl-phenyl)-1-(2-chloro-pyridin-4-ylmethyl)-5,5-dimethyl-imidazolidine-2,4-dione peut être préparé comme indiqué dans le Schéma Général ci-dessus.

## Revendications

1. Produits de formule (Ia): 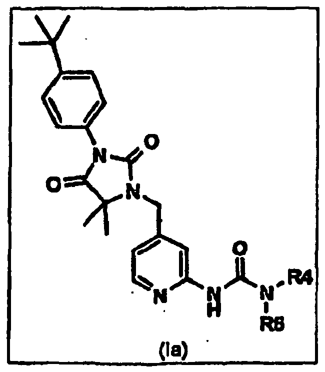 dans laquelle les radicaux NR4R5 sont choisis parmi les radicaux suivants nommés ex 9 à ex 31 : 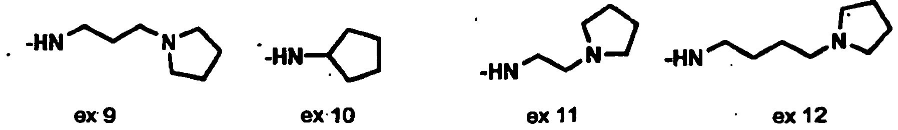 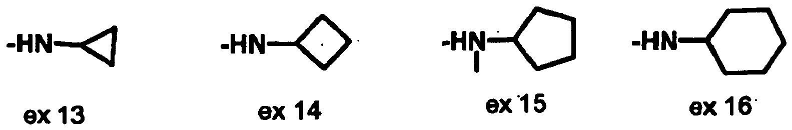 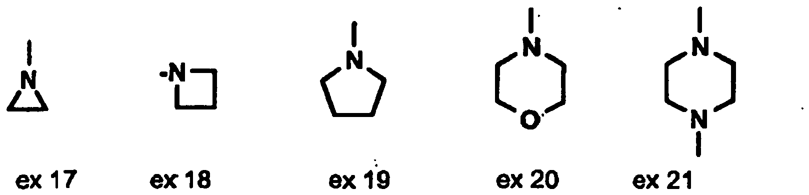 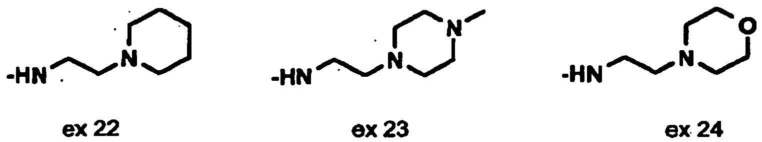 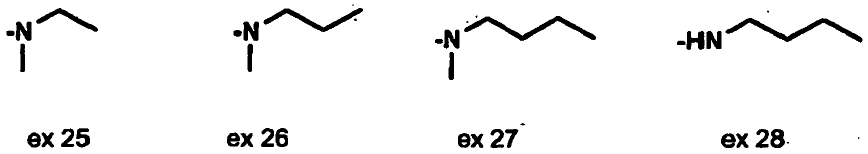 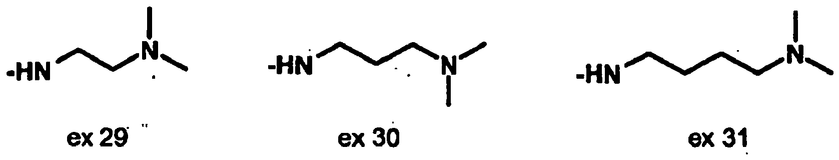 lesdits produits de formule (Ia) étant sous toutes les formes isomères possibles racémiques, énantiomères et diastérêo-isomères, ainsi que les sels d'addition avec les acides minéraux et organiques ou avec les bases minérales et organiques desdits produits de formule (Ia).

2. A titre de médicaments, les produits de formule (Ia) telle que définie à la revendication 1 , lesdits produits de formule (Ia) étant sous toutes les formes isomères possibles racémiques, ériantiomères et diastéréo-isomères, ainsi que les sels d'addition avec les acides minéraux et organiques ou avec les bases minérales et organiques pharmaceutiquement acceptables desdits produits de formule (Ia).

3. Les compositions pharmaceutiques contenant à titre de principe actif l'un au moins des médicaments tels que définis à la revendication 2.

4. Compositions pharmaceutiques telles que définies à la revendication précédente contenant en plus des principe actifs d'autres médicaments de chimiothérapie contre le cancer.

5. Compositions pharmaceutiques selon l'une quelconque des revendications 3 et 4 **caractérisées en ce qu'**elles sont utilisées comme médicaments, en particulier pour la chimiothérapie de cancers.

6. Utilisation de produits de formule (Ia) telle que définie à l'une quelconque des revendications précédentes ou de sels pharmaceutiquement acceptables desdits produits de formule (Ia) pour la préparation d'un médicament destiné à prévenir ou traiter une maladie appartenant au groupe suivant: désordres de la prolifération de vaisseaux sanguins, désordres fibrotiques, désordres de la prolifération de cellules mésangiales, désordres métaboliques, allergies, asthme, thromboses, maladies du système nerveux, rétinopathies, psoriasis, arthrite rhumatoide, diabètes, dégénération musculaire, maladies en oncologie, cancers.

7. Utilisation de produits de formule (Ia) telle que définie à la revendication précédente ou de sels pharmaceutiquement acceptables desdits produits de formule (Ia) pour la préparation d'un médicament destiné à traiter des cancers.

8. Utilisation de produits de formule (Ia) selon la revendication précédente dans laquelle la maladie à traiter est un cancer de tumeurs solides ou liquides.

9. Utilisation de produits de formule (Ia) selon la 5 revendication précédente dans laquelle la maladie à traiter est un cancer résistant aux agents cytotoxiques.

10. Utilisation de produits de formule (Ia) telle que définie à l'une quelconque des revendications 6 à 9 ou de sels pharmaceutiquement acceptables desdits produits de formule (Ia) pour la préparation d'un médicament destiné à traiter des cancers parmi lesquels les cancers du sein, de l'estomac, du colon, des poumons,
des ovaires, de l'utérus, du cerveau, du rein, du larynx,
du système lymphatique, de la thyroïde, du tractus uro-génital, du tractus incluant vésicule et prostate, du cancer des os, du pancréas, les mélanomes.

11. Utilisation de produits de formule (Ia) selon la revendication précédente dans laquelle la maladie à traiter est un cancer du sein, du colon ou des poumons.

12. Utilisation de produits de formule. (Ia) telle que définie à l'une quelconque des revendications 6 à 11 ou de sels pharmaceutiquement acceptables desdits produits de formule (Ia) pour la préparation d'un médicament destiné à la chimiothérapie de cancers.

13. Utilisation de produits de formule (Ia) telle que définie à l'une quelconque des revendications 6 à 12 ou de sels pharmaceutiquement acceptables desdits produits de formule (Ia) pour la préparation de médicaments destinés à la chimiothérapie de cancers utilisés seuls ou en association.

14. Utilisation de produits de formule (Ia) telle que définie à l'une quelconque des revendications 6 à 13 ou de sels pharmaceutiquement acceptables desdits produits de formule (Ia) pour la préparation de médicaments destinés à être utilisés seuls ou en association avec chimiothérapie ou radiothérapie ou alternativement en association avec d'autres agents thérapeutiques.

15. Utilisation de produits de formule (Ia) selon la revendication précédente dans laquelle les agents 5 thérapeutiques peuvent être des agents anti-tumoraux utilisés communément.

16. Produits de formule (Ia) tels que définis en
revendication 1 comme inhibiteurs de IGF1R, lesdits produits de formule (Ia) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (Ia).

## Claims

1. Products of formula (Ia) : 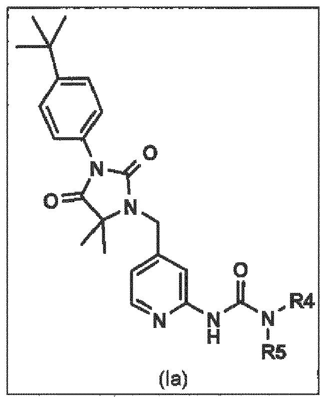 in which the radicals NR4R5 are chosen from the following radicals named ex 9 to ex 31 : 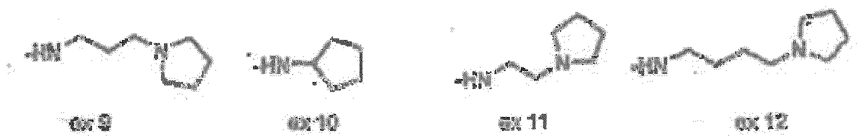 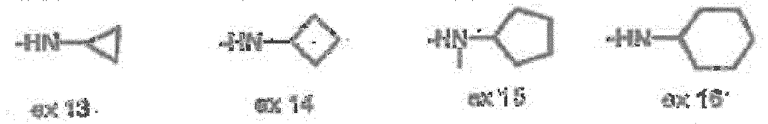 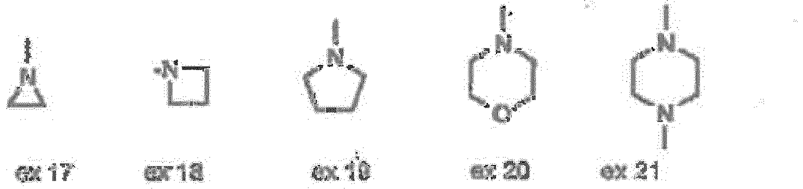 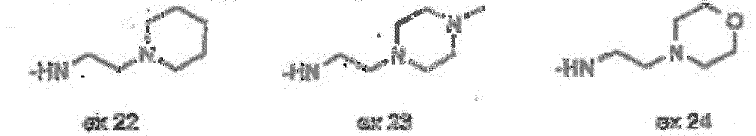 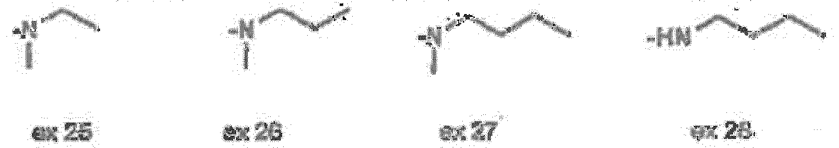 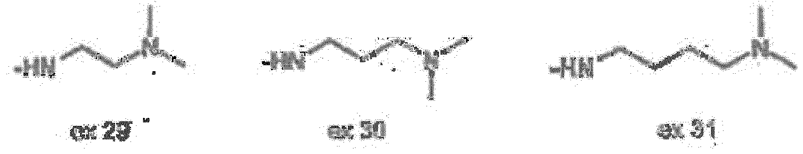 the said products of formula (Ia) being in any possible racemic, enantiomeric or diastereoisomeric isomer form, and also the addition salts with mineral and organic acids or with mineral and organic bases of the said products of formula (Ia).

2. As medicaments, the products of formula (Ia) as defined in Claim 1, the said products of formula (Ia) being in any possible racemic, enantiomeric or diastereoisomeric isomer form, and also the addition salts with pharmaceutically acceptable mineral and organic acids or with pharmaceutically acceptable mineral and organic base of said products of formula (Ia).

3. Pharmaceutical compositions containing, as active principle, at least one of the medicaments as defined in Claim 2.

4. Pharmaceutical compositions as defined in the preceding claim, also containing active principles of other chemotherapy medicaments for combating cancer.

5. Pharmaceutical compositions according to either of Claims 3 and 4, **characterized in that** they are used as medicaments, in particular for cancer chemotherapy.

6. Use of products of formula (Ia) as defined in any one of the preceding claims or of pharmaceutically acceptable salts of said products of formula (Ia), for the preparation of a medicament for reventing or treating a disease belonging to the following group: disorders of blood vessel proliferation, fibrotic disorders, disorders of mesangial cell proliferation, metabolic disorders, allergies, asthma, thrombosis, diseases of the nervous system, retinopathy, psoriasis, rheumatoid arthritis, diabetes, muscle degeneration, oncology diseases and cancers.

7. Use of products of formula (Ia) as defined in the preceding claim or of pharmaceutically acceptable salts of said products of formula (Ia) for the preparation of a medicament for treating cancers.

8. Use of products of formula (Ia) according to the preceding claim, in which the disease to be treated is a cancer of solid or liquid tumours.

9. Use of products of formula (Ia) according to the preceding claim, in which the disease to be treated is a cancer that is resistant to cytotoxic agents.

10. Use of products of formula (Ia) as defined in any one of Claims 6 to 9 or of pharmaceutically acceptable salts of said products of formula (Ia) for the preparation of a medicament for treating cancers including breast cancer, stomach cancer, cancer of the colon, lung cancer, cancer of the ovaries, cancer of the uterus, brain cancer, cancer of the kidney, cancer of the larynx, cancer of the lymphatic system, cancer of the thyroid, cancer of the urogenital tract, cancer of the tract including the seminal vesicle and prostate, bone cancer, cancer of the pancreas and melanomas.

11. Use of products of formula (Ia) according to the preceding claim, in which the disease to be treated is a breast cancer, cancer of the colon or lung cancer.

12. Use of products of formula (Ia) as defined in any one of Claims 6 to 11, or of pharmaceutically acceptable salts of said products of formula (Ia), for the preparation of a medicament for cancer chemotherapy.

13. Use of products of formula (Ia) as defined in any one of Claims 6 to 12 or of pharmaceutically acceptable salts of said products of formula (Ia), for the preparation of medicaments for cancer chemotherapy, used alone or in combination.

14. Use of products of formula (Ia) as defined in any one of Claims 6 to 13 or of pharmaceutically acceptable salts of said products of formula (Ia) for the preparation of medicaments to be used alone or in combination with a chemotherapy or radiotherapy, or alternatively in combination with other therapeutic agents.

15. Use of products of formula (Ia) according to the preceding claim, in which the therapeutic agents may be commonly used antitumour agents.

16. Products of formula (Ia) as defined in Claim 1, as IGF1R, inhibitors, said products of formula (Ia) being in any possible racemic, enantiomeric or diastereoisomeric isomer form, and also the addition salts with pharmaceutically acceptable mineral and organic acids or with pharmaceutically acceptable mineral and organic bases of said products of formula (Ia).

## Patentansprüche

1. Produkte der Formel (Ia) 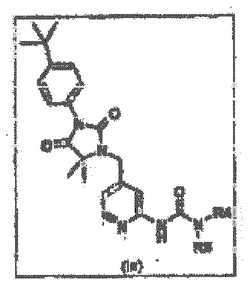 in der
die NR4R5-Reste aus den folgenden Radikalen mit den Bezeichnungen Bsp. 9 bis Bsp. 31 ausgewählt sind: 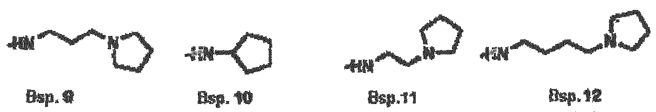 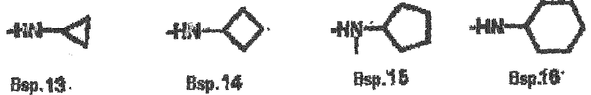 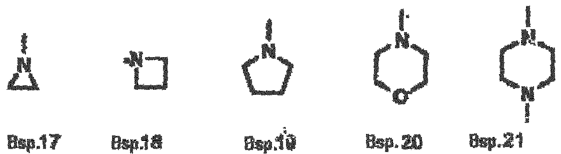 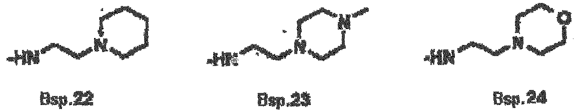 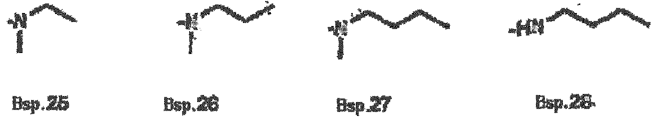 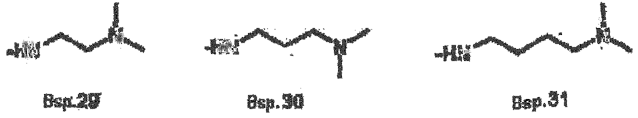 wobei die Produkte der Formel (Ia) in allen möglichen racemischen, enantiomeren und diastereomeren Isoformen vorliegen, sowie die Additionssalze der Produkte der Formel (Ia) mit den anorganischen und organischen Säuren bzw. mit den anorganischen und organischen Basen.

2. Produkte der Formel (Ia) wie in Anspruch 1 definiert, wobei die Produkte der Formel (Ia) in allen möglichen racemischen, enantiomeren und diastereomeren Isoformen vorliegen, sowie die Additionssalze der Produkte der Formel (Ia) mit den anorganischen und organischen Säuren bzw. mit den anorganischen und organischen Basen, als Arzneimittel.

3. Pharmazeutische Zusammensetzungen, die mindestens eins der Arzneimittel wie in Anspruch 2 definiert als Wirkprinzip entfalten.

4. Pharmazeutische Zusammensetzungen wie in dem vorhergehenden Anspruch definiert, die zusätzlich Mirkprinzipien von anderen Chemotherapeutika gegen Krebs enthalten.

5. Pharmazeutische Zusammensetzungen nach einem der Ansprüche 3 und 4, **dadurch gekennzeichnet, dass** sie als Arzneimittel eingesetzt werden, insbesondere für die Chemotherapie von Krebs.

6. Verwendung von Produkten der Formel (Ia) wie in einem der vorhergehenden Ansprüche definiert oder von pharmazeutisch unbedenklichen Salzen der Produkte der Formel (Ia) für die Herstellung eines Arzneimittels zur Vorbeugung oder Behandlung einer Krankheit aus der folgenden Gruppe: Blutgefäßproliferationsstörungen, fibrotische Störungen, Störungen der Mesangialzellproliferation, Stoffwechselstörungen, Allergien, Asthma, Thrombosen, Erkrankungen des Nervensystems, Retinopathien, Psoriasis, rheumatoide Arthritis, Diabetes, Muskeldegeneration, onkologische Krankheiten, Krebs.

7. Verwendung von Produkten der Formel (Ia) wie in dem vorhergehenden Anspruch definiert oder von pharmazeutisch unbedenklichen Salzen der Produkte der Formel (Ia) zur Herstellung eines Medikaments, das zur Behandlung von Krebs bestimmt ist.

8. Verwendung von Produkten der Formel (Ia) nach dem vorhergehenden spruch, wobei es sich bei der zu behandelnden Krankheit um Krebs in Form von festen oder flüssigen Tumoren handelt.

9. Verwendung von Produkten der Formel (Ia) nach dem vorhergehenden spruch, wobei es sich bei der zu behandelnden Krankheit um Krebs, der gegen cytotoxische Mittel resistent ist, handelt.

10. Verwendung von Produkten der Formel (Ia) wie in einem der Ansprüche 6 bis 9 definiert oder von pharmazeutisch unbedenklichen Salzen der Produkte der Formel (Ia) zur Herstellung eines Arzneimittels, das zur Behandlung von Krebs bestimmt ist, darunter Brustkrebs, Magenkrebs, Dickdarmkrebs, Lungenkrebs, Eierstockkrebs, Gebärmutterkrebs, Hirnkrebs, Nierenkrebs, Kehlkopfkrebs, Lymphsystemkrebs, Schilddrüsenkrebs, Krebs des Urogenitaltrakts, Krebs des Trakts, der Samenblase und Prostata beinhaltet, Knochenkrebs, Bauchspeicheldrüsenkrebs, Melanome.

11. Verwendung von Produkten der Formel (Ia) nach dem vorhergehenden Anspruch, wobei es sich bei der zu behandelnden Krankheit um Brustkrebs, Dickdarmkrebs oder Lungenkrebs handelt.

12. Verwendung von Produkten der Formel (Ia) wie in einem der Ansprüche 6 bis 11 definiert, oder von pharmazeutisch unbedenklichen Salzen der Produkte der Formel (Ia) zur Herstellung eines Arzneimittels, das für die Chemotherapie von Krebs bestimmt ist.

13. Verwendung von Produkten der Formel (Ia) wie in einem der Ansprüche 6 bis 12 definiert, oder von pharmazeutisch unbedenklichen Salzen der Produkte der Formel (Ia) zur Herstellung von Arzneimitteln, die für die Chemotherapie von Krebs bestimmt sind, die allein oder in Kombination verwendet werden.

14. Verwendung von Produkten der Formel (Ia) wie in einem der Ansprüche 6 bis 13 definiert, oder von pharmazeutisch unbedenklichen Salzen der Produkte der Formel (Ia) zur Herstellung von Arzneimitteln, die allein oder in Kombination mit Chemotherapie oder Radiotherapie oder alternativ in Kombination mit anderen Therapeutika eingesetzt werden.

15. Verwendung von Produkten der Formel (Ia) nach dem vorhergehenden Anspruch, wobei es sich bei den Therapeutika um üblicherweise verwendete Antitumormittel handeln kann.

16. Produkte der Formel (Ia) wie in Anspruch 1 definiert als IGF1R-Hemmer, wobei die Produkte der Formel (Ia) in allen möglichen racemischen, enantiomeren und diastereomeren Isomerformen sowie als pharmazeutisch unbedenkliche Salze der Produkte der Formel (Ia) mit den anorganischen und organischen Säuren bzw. mit den anorganischen und organischen Basen vorliegen.
